(19) Europäisches Patentamt
European Patent Office
Office européen des brevets

(11) EP 3 509 116 A1

(12) **EUROPEAN PATENT APPLICATION**
published in accordance with Art. 153(4) EPC

(43) Date of publication:
**10.07.2019 Bulletin 2019/28**

(21) Application number: **17846389.9**

(22) Date of filing: **28.08.2017**

(51) Int Cl.:
***H01L 51/44*** *(2006.01)* ***H01G 9/20*** *(2006.01)*

(86) International application number:
**PCT/JP2017/030699**

(87) International publication number:
**WO 2018/043384 (08.03.2018 Gazette 2018/10)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **31.08.2016 JP 2016168769**

(71) Applicant: **FUJIFILM Corporation**
**Tokyo 106-8620 (JP)**

(72) Inventors:
- **SHIROKANE, Kenji**
**Ashigarakami-gun**
**Kanagawa 258-8577 (JP)**
- **SATOU, Hirotaka**
**Ashigarakami-gun**
**Kanagawa 258-8577 (JP)**
- **ISE, Toshihiro**
**Ashigarakami-gun**
**Kanagawa 258-8577 (JP)**

(74) Representative: **Hoffmann Eitle**
**Patent- und Rechtsanwälte PartmbB**
**Arabellastraße 30**
**81925 München (DE)**

(54) **PHOTOELECTRIC CONVERSION ELEMENT AND SOLAR BATTERY**

(57) Provided is a photoelectric conversion element, including a first electrode that has a photosensitive layer containing a light absorbing agent on a conductive support, in which the light absorbing agent contains a compound having a perovskite-type crystal structure that has an organic cation represented by Formulas (1) and (2), a cation of a metal atom, and an anion; and a solar cell which is formed of this photoelectric conversion element.

Formula (1) $C(R^1)(R^2)(R^3)\text{-}N(R^{1a})_3^+$

Formula (2) $R^4\text{-}NR^{2a}{}_3^+$

$R^1$, $R^2$, $R^3$, and $R^4$ represent specific substituent that may be different from each other. $R^{1a}$ and $R^{2a}$ represent a hydrogen atom or a specific substituent.

FIG. 1

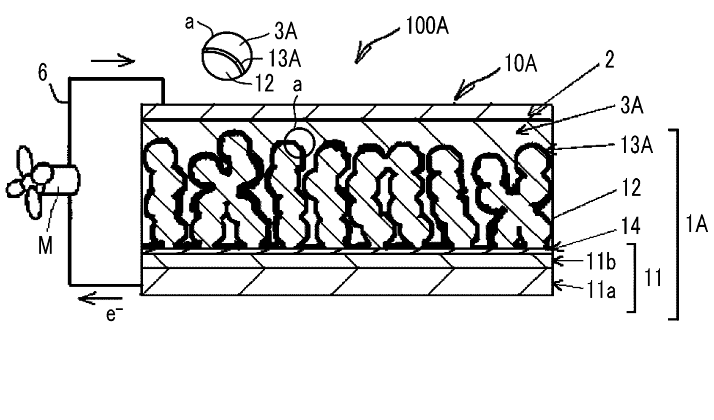


EP 3 509 116 A1

## Description

## BACKGROUND OF THE INVENTION

1. Field of the Invention

The present invention relates to a photoelectric conversion elements and a solar cell.

2. Description of the Related Art

Photoelectric conversion elements are used in a variety of optical sensors, copiers, solar cells, and the like. Practical use of solar cells as cells using non-exhaustible solar energy is proceeding. Among these, research and development of dye sensitized solar cells, in which an organic dye, a Ru bipyridyl complex, or the like is used as a sensitizer, are actively in progress, and the photoelectric conversion efficiency thereof reaches approximately 11%.

Meanwhile, in recent years, there have been reported research results indicating that solar cells using a metal halide as a compound having a perovskite-type crystal structure are capable of achieving relatively high photoelectric conversion efficiency, and the solar cells attract attention. For example, a solar cell formed of a metal halide represented by $CH_3NH_3PbI_2Cl$ as a light absorbing agent is described in Science, 2012, vol. 338, p. 643-647.

In addition, a solar cell formed of a layered perovskite represented by $(PEA)_2(MA)_2[Pb_3I_{10}]$ is described in Angew. Chem. Int. Ed. 2014, 53, p. 11232-11235. The PEA referred herein represents $C_6H_5(CH_2)_2NH_3^+$, and the MA referred herein represents $CH_3NH_3^+$.

Furthermore, a perovskite solar cell having a three-layered structure of a $TiO_2$ layer, a $ZrO_2$ layer, and a layer composed of $(5\text{-AVA})_x(MA)_{1-x}PbI_3$ is described in Science, 2014, vol. 345, p.295. The 5-AVA referred herein represents a 5-aminovaleric acid cation, and the MA referred herein represents a methylammonium cation.

## SUMMARY OF THE INVENTION

As described above, in regard to a photoelectric conversion element formed of a compound having a perovskite-type crystal structure (hereinafter referred to as "perovskite compound"), certain achievements in improving a photoelectric conversion efficiency has been experimentally obtained.

However, there is a problem that the perovskite compound is easily damaged under a high-humidity environment. That is, in a photoelectric conversion element or a solar cell which uses the perovskite compound in a photosensitive layer, the photoelectric conversion efficiency greatly deteriorates over time particularly under a high-humidity environment in many cases. It is perceived that the reason for this is because a crystal structure of the perovskite compound is damaged under a high-humidity environment, resulting in changes in a surface state of the photosensitive layer, and thus adhesiveness with an adjacent layer deteriorates.

An object of the present invention is to provide a photoelectric conversion element including a photosensitive layer containing a perovskite compound, in which, even in a case of being used under a high-humidity environment, a crystal structure of a photosensitive layer is unlikely to be damaged, and a deterioration in photoelectric conversion efficiency over time is efficiently suppressed. Another object of the present invention is to provide a solar cell formed of the above photoelectric conversion element.

The inventors of the present invention have found that, in a photoelectric conversion element including a photosensitive layer in which at least a part of the layer is composed of a perovskite compound, by allowing a light-absorptive material of the photosensitive layer to contain at least two kinds of organic ammonium ions having a specific structure as a cation composing the perovskite-type crystal structure, a crystal structure of the photosensitive layer is unlikely to be damaged (which is decomposition of the crystal structure), and a deterioration in photoelectric conversion efficiency over time can be efficiently suppressed ever under a high-humidity environment.

The present invention has been completed through investigation based on these findings.

That is, the above-described problem of the present invention has been solved by the following means.

[1] A photoelectric conversion element, comprising: a first electrode that includes a photosensitive layer containing a light absorbing agent on a conductive support; and a second electrode that is opposite to the first electrode,
in which the light absorbing agent contains a compound having a perovskite-type crystal structure that has an organic cation, a cation of a metal atom, and an anion, and
the organic cation includes an organic cation represented by Formula (1) and an organic cation represented by Formula (2).

Formula (1) 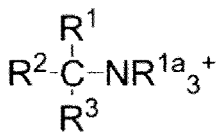

In Formula (1), $R^1$ and $R^2$ represent a group selected from a substituent group Z below, $R^3$ represents a hydrogen atom or a group selected from the substituent group Z below, and $R^{1a}$ represents a hydrogen atom or a substituent, provided that not all of $R^1$, $R^2$ and $R^3$ are acidic groups.
[Substituent group Z]: a group represented by Formula (1a), an aryl group, a cycloalkyl group, a heteroaryl group, an aliphatic heterocyclic group, an alkenyl group, an alkynyl group, and an acidic group

Formula (1a) $R^{1b}—(CR^{1c}_2)_n—*$

In Formula (1a), $R^{1b}$ represents a methyl group, a silyl group, a cycloalkyl group, an aryl group, a heteroaryl group, an aliphatic heterocyclic group, a halogen atom, or an acidic group, $R^{1c}$ represents a hydrogen atom or represents a substituent other than an alkenyl group and other than an alkynyl group, n represents integer of 3 to 15, and * represents a binding site with a carbon atom represented in Formula (1).

Formula (2) $R^4\text{-}NR^{2a}{}_3{}^+$

In Formula (2), $R^4$ represents a methyl group, an ethyl group, or a group represented by Formula (2a), and $R^{2a}$ represents a hydrogen atom, an alkyl group, a cycloalkyl group, an alkenyl group, an alkynyl group, an aryl group, a heteroaryl group, or an aliphatic heterocyclic group.

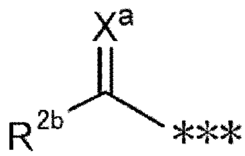

Formula (2a)

In Formula (2a), $X^a$ represents $NR^{2c}$, an oxygen atom, or a sulfur atom, $R^{2b}$ and $R^{2c}$ represent a hydrogen atom or a substituent, and *** represents a binding site with a nitrogen atom represented in Formula (2).
[2] The photoelectric conversion element according to [1], in which $R^1$ and $R^2$ represent a group selected from a group represented by Formula (1a), an aryl group, a cycloalkyl group, a heteroaryl group, an aliphatic heterocyclic group, an alkenyl group, and an alkynyl group, and $R^3$ represents a hydrogen atom or a group selected from the substituent group Z.
[3] The photoelectric conversion element according to [1] or [2], in which all of $R^1$, $R^2$, and $R^3$ represent a group selected from a group represented by Formula (1a), an aryl group, a cycloalkyl group, a heteroaryl group, an aliphatic heterocyclic group, an alkenyl group, and an alkynyl group.
[4] The photoelectric conversion element according to [3], in which $R^1$ and $R^2$ represent a group selected from a group represented by Formula (1a), an alkenyl group, and an alkynyl group.
[5] The photoelectric conversion element according to [4], in which $R^1$ and $R^2$ represent a group represented by Formula (1a), and $R^{1b}$ in $R^1$ represents a methyl group, a silyl group, a cycloalkyl group, an aryl group, or a halogen atom, and $R^{1b}$ in $R^2$ represents an acidic group.
[6] The photoelectric conversion element according to [5], in which $R^{1b}$ in $R^1$ represents a methyl group, a silyl group, or a cycloalkyl group, and $R^{1b}$ in $R^2$ represents an acidic group.
[7] The photoelectric conversion element according to [5] or [6], in which all of $R^1$, $R^2$, and $R^3$ represent a group represented by Formula (1a).
[8] A solar cell which is formed of the photoelectric conversion element according to any one of [1] to [7].

In this specification, parts of respective formulae may be expressed as a rational formula for understanding of chemical structures of compounds. According to this, in the respective formulae, partial structures are called (substituent) groups, ions, atoms, and the like, but in this specification, the partial structures may represent element groups or elements

which constitute (substituent) groups or ions represented by the formulae in addition to the (substituent) groups, the ions, the atoms, and the like.

In this specification, with regard to expression of compounds (including a complex and a dye), the expression is also used to indicate salts of the compounds and ions of the compounds in addition to the compounds. In addition, with regard to compounds for which substitution or non-substitution is not specified, the compounds also include compounds which have any substituent in a range not imparting a desired effect. This is also true of substituents, linking groups, and the like (hereinafter, referred to as "substituents and the like").

In this specification, in a case where a plurality of substituents or linking groups (hereinafter referred to as substituent and the like) expressed using specific symbols are present, or in a case where a plurality of substituents and the like are defined at the same time, the respective substituents and the like may be identical to or different from each other unless otherwise stated. This is also true of definition of the number of substituents and the like. In addition, in a case of approaching each other (particularly, in a case of being close to each other), the plurality of substituents and the like may be bonded to each other to form a ring unless otherwise stated. In addition, rings, for example, aliphatic rings, aromatic rings, and hetero rings may be additionally fused together to form a fused ring.

In this specification, numerical ranges represented by using "to" include ranges including numerical values before and after "to" as the lower limit and the upper limit.

In the photoelectric conversion element and the solar cell of the present invention, in the photosensitive layer containing the perovskite compound, a crystal structure of the layer is unlikely to be damaged even under a high-humidity environment, and a deterioration in photoelectric conversion efficiency over time at the time of being used under a high-humidity environment can be efficiently suppressed.

The above and other features and advantages of the present invention will become more apparent from the following description with reference to the accompanying drawings as appropriate.

## BRIEF DESCRIPTION OF THE DRAWINGS

Fig. 1 is a cross-sectional view (including an enlarged view of a circle part in the layer) schematically illustrating a preferred aspect of a photoelectric conversion element obtained by a manufacturing method of the present invention.

Fig. 2 is a cross-sectional view schematically illustrating another preferred aspect of a photoelectric conversion element obtained by a manufacturing method of the present invention, which has a thick photosensitive layer.

Fig. 3 is a cross-sectional view schematically illustrating still another preferred aspect of a photoelectric conversion element obtained by a manufacturing method of the present invention.

Fig. 4 is a cross-sectional view schematically illustrating further still another preferred aspect of a photoelectric conversion element obtained by a manufacturing method of the present invention.

Fig. 5 is a cross-sectional view schematically illustrating further still another preferred aspect of a photoelectric conversion element obtained by a manufacturing method of the present invention.

Fig. 6 is a cross-sectional view schematically illustrating further still another preferred aspect of a photoelectric conversion element obtained by a manufacturing method of the present invention.

## DESCRIPTION OF THE PREFERRED EMBODIMENTS

[Photoelectric conversion element]

A photoelectric conversion element according to the embodiment of the invention includes a first electrode that has a form in which a photosensitive layer is provided on a conductive support, and a second electrode that is opposite to the first electrode. A case where the first electrode and the second electrode are opposed to each other means both aspect in which the first electrode and the second electrode are laminated in a state of being in contact with each other, and aspect in which the first electrode and the second electrode are laminated via another layer (that is, an aspect in which the first electrode and the second electrode are provided so as to be opposed to each other with another layer sandwiched therebetween). In addition, in the first electrode, the photosensitive layer is disposed on a side closer to the second electrode than the conductive support.

The photosensitive layer contains a light absorbing agent having two specific kinds of organic cations, a metal cation, and an anion, in which at least a part of this light absorbing agent has a perovskite-type crystal structure. The anion may be an anionic atom or an anionic atomic group. In a case where a portion that is not the perovskite-type crystal structure is present in the light absorbing agent, in this portion that is not the perovskite-type crystal structure, a state where the cation and the anion are bonded by an ionic bond can be adopted.

In the present invention, the case in which the photosensitive layer is provided on the conductive support means

an aspect in which the photosensitive layer is provided to be in contact with (directly provided on) a surface of the conductive support, and an aspect in which the photosensitive layer is provided on an upper side of the surface of the conductive support through another layer.

In the aspect in which the photosensitive layer is provided on the upper side of the surface of the conductive support through another layer, as the other layer that is provided between the conductive support and the photosensitive layer, there is no particular limitation as long as the other layer does not deteriorate a cell performance of a solar cell. Examples thereof include a porous layer, a blocking layer, an electron transport layer, a hole transport layer, and the like.

In the present invention, examples of the aspect in which the photosensitive layer is provided on the upper side of the surface of the conductive support through another layer includes an aspect in which the photosensitive layer is provided on the surface of the porous layer in a thin film shape (refer to Fig. 1) or in a thick film shape (refer to Figs. 2 and 6), an aspect in which the photosensitive layer is provided on a surface of the blocking layer in a thin film shape or a thick film shape (refer to Fig. 3), an aspect in which the photosensitive layer is provided on a surface of the electron transport layer in a thin film shape or a thick film shape (refer to Fig. 4), and an aspect in which the photosensitive layer is provided on a surface of the hole transport layer in a thin film shape or a thick film shape (refer to Fig. 5). The photosensitive layer may be provided in a linear shape or in a dispersed pattern, but is preferably provided in a film shape.

In the photoelectric conversion element according to the embodiment of the invention, the photosensitive layer contains the light absorbing agent having two specific kinds of organic cations, a cation of a metal atom, and an anion, in which at least a part of this light absorbing agent is composed of a perovskite compound. With such a configuration, in the photoelectric conversion elements, changes in a form of the photosensitive layer under a high-humidity environment (damage on a crystal structure) can be suppressed, and a deterioration in photoelectric conversion efficiency can be efficiently suppressed.

In the photoelectric conversion element of the embodiment of the invention, a configuration other than a configuration defined in the present invention is not particularly limited, and it is possible to employ a configuration that is known with respect to the photoelectric conversion element and a solar cell. Respective layers, which constitute the photoelectric conversion element of the embodiment of the invention, are designed in correspondence with the purposes thereof, and may be formed, for example, in a monolayer or multilayers. For example, the porous layer may be provided between the conductive support and the photosensitive layer (refer to Figs. 1, 2, and 6).

Hereinafter, description will be given of preferred aspects of the photoelectric conversion element of the embodiment of the invention.

In Figs. 1 to 6, the same reference numeral represents the same constituent element (member).

Furthermore, in Figs. 1, 2, and 6, the size of fine particles which form a porous layer 12 is illustrated in a highlighted manner. These fine particles are preferably packed with each other (are vapor-deposited or in close contact with each other) in the horizontal direction and the vertical direction with respect to a conductive support 11 to form a porous structure.

In this specification, simple description of "photoelectric conversion element 10" represents photoelectric conversion elements 10A to 10F unless otherwise stated. The same applies to a system 100 and a first electrode 1. In addition, simple description of "photosensitive layer 13" represents photosensitive layers 13A to 13C unless otherwise stated. Similarly, description of "hole transport layer 3" represents hole transport layers 3A and 3B unless otherwise stated.

Examples of a preferred aspect of the photoelectric conversion element of the embodiment of the invention include the photoelectric conversion element 10A illustrated in Fig. 1. A system 100A illustrated in Fig. 1 is a system in which the photoelectric conversion element 10A is applied to use of a cell that allows operation means M (for example, an electric motor) to operate by an external circuit 6.

The photoelectric conversion element 10A includes a first electrode 1A, a second electrode 2, and a hole transport layer 3A between the first electrode 1A and the second electrode 2.

The first electrode 1A includes a conductive support 11 including a support 11a and a transparent electrode 11b, a porous layer 12, and a photosensitive layer 13A which contains a perovskite compound and is provided on a surface of the porous layer 12 as schematically illustrated in enlarged cross-sectional region a in which a cross-sectional region a is enlarged in Fig. 1. In addition, a blocking layer 14 is provided on the transparent electrode 11b, and the porous layer 12 is formed on the blocking layer 14. As described above, in the photoelectric conversion element 10A including the porous layer 12, it is assumed that a surface area of the photosensitive layer 13A increases, and thus charge separation and charge migration efficiency are improved.

A photoelectric conversion element 10B illustrated in Fig. 2 schematically illustrates a preferred aspect in which the photosensitive layer 13A of the photoelectric conversion element 10A illustrated in Fig. 1 is provided to be thick. In the photoelectric conversion element 10B, the hole transport layer 3B is provided to be thin. The photoelectric conversion element 10B is different from the photoelectric conversion element 10A illustrated in Fig. 1 in the film thickness of the photosensitive layer 13B and the hole transport layer 3B, but the photoelectric conversion element 10B has the same configuration as that of the photoelectric conversion element 10A except for the difference.

The photoelectric conversion element 10C illustrated in Fig. 3 schematically illustrates another preferred aspect

of the photoelectric conversion element of the embodiment of the invention. The photoelectric conversion element 10C is different from the photoelectric conversion element 10B illustrated in Fig. 2 in that the porous layer 12 is not provided, but the photoelectric conversion element 10C has the same configuration as that of the photoelectric conversion element 10B except for the difference. That is, in the photoelectric conversion element 10C, the photosensitive layer 13C is formed on the surface of the blocking layer 14 in a thick film shape. In the photoelectric conversion element 10C, the hole transport layer 3B may be provided to be thick in the same manner as in the hole transport layer 3A.

The photoelectric conversion element 10D illustrated in Fig. 4 schematically illustrates still another preferred aspect of the photoelectric conversion element of the embodiment of the invention. The photoelectric conversion element 10D is different from the photoelectric conversion element 10C illustrated in Fig. 3 in that an electron transport layer 15 is provided instead of the blocking layer 14, but the photoelectric conversion element 10D has the same configuration as that of the photoelectric conversion element 10C except for the difference. The first electrode 1D includes the conductive support 11, and the electron transport layer 15 and the photosensitive layer 13C which are sequentially formed on the conductive support 11. The photoelectric conversion element 10D is preferable when considering that the respective layers can be formed from an organic material. According to this, the productivity of the photoelectric conversion element is improved, and thickness reduction or flexibilization becomes possible.

The photoelectric conversion element 10E illustrated in Fig. 5 schematically illustrates still another preferred aspect of the photoelectric conversion element of the embodiment of the invention. A system 100E including the photoelectric conversion element 10E is a system that is applied to use of a cell in the same manner as in the system 100A. The photoelectric conversion element 10E includes a first electrode 1E, a second electrode 2, and an electron transport layer 4 that is provided between the first electrode 1E and the second electrode 2. The first electrode 1E includes the conductive support 11, and a hole transport layer 16 and the photosensitive layer 13C which are formed on the conductive support 11 in this order. The photoelectric conversion element 10E is preferable when considering that respective layers can be formed from an organic material in the same manner as in the photoelectric conversion element 10D.

A photoelectric conversion element 10F illustrated in Fig. 6 schematically illustrates still another preferred aspect of the photoelectric conversion element of the embodiment of the invention. The photoelectric conversion element 10F is different from the photoelectric conversion element 10B illustrated in Fig. 2 in that the hole transport layer 3B is not provided, but the photoelectric conversion element 10F has the same configuration as that of the photoelectric conversion element 10B except for the difference.

In the present invention, a system 100 to which the photoelectric conversion element 10 is applied functions as a solar cell as follows.

Specifically, in the photoelectric conversion element 10, light that is transmitted through the conductive support 11, or the second electrode 2 and is incident to the photosensitive layer 13 excites a light absorbing agent. The excited light absorbing agent includes high-energy electrons and can emit the electrons. The light absorbing agent, which emits high-energy electrons, becomes an oxidized substance (cation).

In the photoelectric conversion elements 10A to 10D and 10F, electrons emitted from the light absorbing agent migrate between a plurality of the light absorbing agents and reach the conductive support 11. The electrons which have reached the conductive support 11 work in the external circuit 6, and then return to the photosensitive layer 13 through the second electrode 2 (and also through the hole transport layer 3 in a case where the hole transport layer 3 is present). The light absorbing agent is reduced by the electrons which have returned to the photosensitive layer 13. Meanwhile, in the photoelectric conversion element 10E, the electrons which have emitted from the light absorbing agent reaches the second electrode 2 from the photosensitive layer 13C through the electron transport layer 4, work in the external circuit 6, and then return to the photosensitive layer 13 through the conductive support 11. The light absorbing agent is reduced by the electrons which have returned to the photosensitive layer 13.

As described above, in the photoelectric conversion element 10, a cycle of excitation of the light absorbing agent and electron migration is repeated, and thus the system 100 functions as a solar cell.

In the photoelectric conversion elements 10A to 10D and 10F, a method of allowing an electron to flow from the photosensitive layer 13 to the conductive support 11 is different depending on presence or absence of the porous layer 12, a kind thereof, and the like. In the photoelectric conversion element 10 of the embodiment of the invention, electron conduction, in which electrons migrate between the light absorbing agents, occurs. Accordingly, in the present invention, in a case where the porous layer 12 is provided, the porous layer 12 can be formed from an insulating substance other than semiconductors in the related art. In a case where the porous layer 12 is formed from a semiconductor, electron conduction, in which electrons migrate at the inside of semiconductor fine particles of the porous layer 12 or between the semiconductor fine particles, also occurs. On the other hand, in a case where the porous layer 12 is formed from an insulating substance, electron conduction in the porous layer 12 does not occur. In a case where the porous layer 12 is formed from the insulating substance, in a case of using fine particles of an aluminum oxide ($Al_2O_3$) as the fine particles of the insulating substance, a relatively high electromotive force ($V_{OC}$) is obtained.

Even in a case where the blocking layer 14 as the other layer is formed from a conductor or a semiconductor, electron conduction in the blocking layer 14 occurs. In addition, even in the electron transport layer 15, electron conduction

occurs.

The photoelectric conversion elements of the embodiment of the invention are not limited to the preferred aspects, and configurations and the like of the respective aspects may be appropriately combined between the respective aspects in a range not departing from the gist of the present invention. For example, as the configuration of the photoelectric conversion element 10C or 10D, the hole transport layer 3B may not be provided, as in the photoelectric conversion element 10F.

In the present invention, materials and respective members which are used in the photoelectric conversion element can be prepared by using a typical method except the light absorbing agent. With regard to the photoelectric conversion element formed of the perovskite compound or the solar cell, for example, it is possible to refer to Science, 2012, vol. 338, p. 643-647; Angew. Chem. Int. Ed. 2014, 53, p. 11232-11235; and Science, 2014, vol. 345, p.295. In addition, it is possible to refer to J. Am. Chem. Soc., 2009, 131 (17), p. 6050-6051 and Science, 338, p. 643 (2012).

In addition, reference can be made to materials and respective members which are used in a dye sensitized solar cell. With regard to dye sensitized solar cells, for example, reference can be made to JP2001-291534A, US4,927,721A, US4,684,537A, US5,084,365A, US5,350,644A, US5,463,057A, US5,525,440A, JP1995-249790A (JP-H7-249790A), JP2004-220974A, and JP2008-135197A.

Hereinafter, preferred aspects of members and compounds that the photoelectric conversion element of the embodiment of the invention comprises will be explained.

<First electrode 1>

The first electrode 1 includes the conductive support 11 and the photosensitive layer 13, and functions as an operation electrode in the photoelectric conversion element 10.

It is preferable that the first electrode 1 has at least one of the porous layer 12, the blocking layer 14, the electron transport layer 15, or the hole transport layer 16 as illustrated in Figs. 1 to 6.

It is preferable that the first electrode 1 includes at least the blocking layer 14 from the viewpoint of short-circuit prevention, and more preferably the porous layer 12 and the blocking layer 14 from the viewpoints of light absorption efficiency and short-circuit prevention.

In addition, it is preferable that the first electrode 1 includes the electron transport layer 15 and the hole transport layer 16 which are formed from an organic material from the viewpoints of an improvement in productivity of the photoelectric conversion element, thickness reduction, and flexibilization.

-Conductive support 11-

The conductive support 11 is not particularly limited as long as the conductive support 11 has conductivity and can support the photosensitive layer 13 and the like. It is preferable that the conductive support 11 has a configuration formed from a conductive material, for example, a metal, or a configuration including the support 11a formed from glass or plastic and the transparent electrode 11b formed on a surface of the support 11a as a conductive film.

Among these, as illustrated in Figs. 1 to 6, it is more preferable that the conductive support 11 has a configuration in which a conductive metal oxide is applied to the surface of the support 11a formed from glass or plastic to form the transparent electrode 11b. Examples of the support 11a formed from plastic include a transparent polymer film described in Paragraph 0153 of JP2001-291534A. As a material that forms the support 11a, it is possible to use ceramic (JP2005-135902A) and a conductive resin (JP2001-160425A) in addition to glass or plastic. As a metal oxide, a tin oxide (TO) is preferable, and an indium-tin oxide (a tin-doped indium oxide; ITO) or a fluorine-doped tin oxide such as a tin oxide doped with fluorine (FTO) is particularly preferable. At this time, the amount of the metal oxide applied is preferably 0.1 to 100 g per 1 $m^2$ of a surface area of the support 11a. In a case of using the conductive support 11, it is preferable that light is incident from a support 11a side.

It is preferable that the conductive support 11 is substantially transparent. In the present invention, "substantially transparent" represents that transmittance of light (having a wavelength of 300 to 1200 nm) is 10% or greater, preferably 50% or greater, and more preferably 80% or greater.

The thickness of the support 11a and the conductive support 11 is not particularly limited and is set to an appropriate thickness. For example, the thickness is preferably 0.01 $\mu$m to 10 mm, more preferably 0.1 $\mu$m to 5 mm, and still preferably 0.3 $\mu$m to 4 mm.

In a case of providing the transparent electrode 11b, the film thickness of the transparent electrode 11b is not particularly limited. For example, the film thickness is preferably 0.01 to 30 $\mu$m, more preferably 0.02 to 25 $\mu$m, and still more preferably 0.025 to 20 $\mu$m.

The conductive support 11 or the support 11a may have a light management function on the surface. For example, the conductive support 11 or the support 11a may include an antireflection film formed by alternately laminating a high-refractive-index and a low-refractive-index oxide film on the surface of the conductive support 11 or the support

11a as described in JP2003-123859A or may have a light guide function as described in JP2002-260746A.

-Blocking layer 14-

In the present invention, as in the photoelectric conversion elements 10A to 10C and 10F, the blocking layer 14 is preferably provided on the surface of the transparent electrode 11b, that is, between the conductive support 11, and the porous layer 12, the photosensitive layer 13, the hole transport layer 3, or the like.

In the photoelectric conversion element, for example, in a case where the photosensitive layer 13 or the hole transport layer 3, and the transparent electrode 11b and the like are electrically connected to each other, a reverse current is generated. The blocking layer 14 plays a role of preventing the reverse current. The blocking layer 14 is also referred to as a "short-circuit prevention layer."

The blocking layer 14 may be allowed to function as a stage that carries the light absorbing agent.

The blocking layer 14 may be provided even in a case where the photoelectric conversion element includes the electron transport layer. For example, in a case of the photoelectric conversion element 10D, the blocking layer 14 may be provided between the conductive support 11 and the electron transport layer 15, and in a case of the photoelectric conversion element 10E, the blocking layer 14 may be provided between the second electrode 2 and the electron transport layer 4.

The material that forms the blocking layer 14 is not particularly limited as long as the material can perform the above-described function, and it is preferable that the material is a material through which visible light is transmitted, and which has insulating properties with respect to the conductive support 11 (transparent electrode 11b) and the like. Specifically, "material having insulating properties with respect to the conductive support 11 (transparent electrode 11b)" represents a compound (n-type semiconductor compound) having a conduction band energy level that is equal to or higher than a conduction band energy level of a material that forms the conductive support 11 (a metal oxide that forms the transparent electrode 11b) and is lower than a conduction band energy level of a material that constitutes the porous layer 12 or a ground state energy level of the light absorbing agent.

Examples of a material that forms the blocking layer 14 include silicon oxide, magnesium oxide, aluminum oxide, calcium carbonate, cesium carbonate, polyvinyl alcohol, polyurethane, and the like. In addition, the material may be a material that is typically used as a photoelectric conversion material, and examples thereof include titanium oxide, tin oxide, zinc oxide, niobium oxide, tungsten oxide, and the like. Among these, titanium oxide, tin oxide, magnesium oxide, aluminum oxide, and the like are preferred.

It is preferable that the film thickness of the blocking layer 14 is 0.001 to 10 μm, more preferably 0.005 to 1 μm, and still more preferably 0.01 to 0.1 μm.

In the present invention, the film thickness of each layer can be measured by observing the cross section of the photoelectric conversion element 10 using a scanning electron microscope (SEM) and the like.

-Porous layer 12-

In the present invention, as in the photoelectric conversion elements 10A, 10B, and 10F, the porous layer 12 is preferably provided on the transparent electrode 11b. In a case where the blocking layer 14 is provided, the porous layer 12 is preferably formed on the blocking layer 14.

The porous layer 12 is a layer that functions as a stage that carries the photosensitive layer 13 on the surface. In a solar cell, so as to increase the light absorption efficiency, it is preferable to increase a surface area of at least a portion that receives light such as solar light, and it is preferable to increase the surface area of the porous layer 12 as a whole.

It is preferable that the porous layer 12 is a fine particle layer that includes pores and is formed through vapor-deposition or close contact of fine particles of a material that forms the porous layer 12. The porous layer 12 may be a fine particle layer that is formed through vapor-deposition of two or more kinds of fine particles. In a case where the porous layer 12 is a fine particle layer that includes pores, it is possible to increase the amount (adsorption amount) of the carried light absorbing agent.

It is preferable to increase the surface area of individual fine particles which constitute the porous layer 12 so as to increase the surface area of the porous layer 12. In the present invention, in a state in which the fine particles are applied to the conductive support 11 and the like, it is preferable that the surface area of the fine particles which form the porous layer 12 is 10 or more times a projected area, and more preferably 100 or more times the projected area. The upper limit thereof is not particularly limited. Typically, the upper limit is approximately 5000 times the projected area. With regard to a particle size of the fine particles which form the porous layer 12, an average particle size, which uses a diameter when converting the projected area into a circle, is preferably 0.001 to 1 μm as primary particles. In a case where the porous layer 12 is formed by using a dispersion of fine particles, the average particle size of the fine particles is preferably 0.01 to 100 μm in terms of an average particle size of the dispersion.

For the material that forms the porous layer 12, there is no particular limitation with respect to conductivity. The material may be an insulating substance (insulating material), a conductive material, or a semiconductor (semi-conductive material).

As the material that forms the porous layer 12, it is possible to use, for example, chalcogenides (for example, an oxide, a sulfide, a selenide, and the like) of metals, compounds having a perovskite-type crystal structure (excluding a perovskite compound that uses a light absorbing agent), oxides of silicon (for example, silicon dioxide, and zeolite), or carbon nanotubes (including carbon nanowires, carbon nanorods, and the like).

The chalcogenides of a metal are not particularly limited, and preferred examples thereof include respective oxides of titanium, tin, zinc, tungsten, zirconium, hafnium, strontium, indium, cerium, yttrium, lanthanum, vanadium, niobium, aluminum, and tantalum, cadmium sulfide, cadmium selenide, and the like. Examples of the crystal structure of the chalcogenides of metals include an anatase-type crystal structure, a brookite-type crystal structure, and a rutile-type crystal structure, and the anatase-type crystal structure and the brookite-type crystal structure are preferable.

The compound having a perovskite-type crystal structure is not particularly limited, and examples thereof include a transition metal oxide and the like. Examples of the transition metal oxide include strontium titanate, calcium titanate, barium titanate, zinc titanate, barium zirconate, barium stannate, zinc zirconate, strontium zirconate, strontium tantalate, potassium niobate, bismuth ferrate, barium strontium titanate, lanthanum barium titanate, calcium titanate, sodium titanate, and bismuth titanate. Among these, strontium titanate, calcium titanate, and the like are preferable.

The carbon nanotubes have a shape obtained by rounding off a carbon film (graphene sheet) into a tubular shape. The carbon nanotubes are classified into a single-walled carbon nanotube (SWCNT) obtained by winding one graphene sheet in a cylindrical shape, a double-walled carbon nanotube (DWCNT) obtained by winding two graphene sheets in a concentric shape, and a multi-walled carbon nanotube (MWCNT) obtained by winding a plurality of graphene sheets in a concentric shape. As the porous layer 12, any carbon nanotubes can be used without any particular limitation.

Among these, as the material that forms the porous layer 12, an oxide of titanium, tin, zinc, zirconium, aluminum, or silicon, or a carbon nanotube is preferable, and titanium oxide or aluminum oxide is more preferable.

The porous layer 12 may be formed from at least one kind of the chalcogenides of metals, the compound having a perovskite-type crystal structure, the oxide of silicon, or the carbon nanotube, or may be formed from a plurality of kinds thereof.

The film thickness of the porous layer 12 is not particularly limited. The thickness is typically in a range of 0.05 to 100 $\mu$m, and preferably in a range of 0.1 to 100 $\mu$m. In a case of being used as a solar cell, the film thickness is preferably 0.1 to 50 $\mu$m, and more preferably 0.2 to 30 $\mu$m.

-Electron transport layer 15-

In the present invention, as in the photoelectric conversion element 10D, the electron transport layer 15 is preferably provided on the surface of the transparent electrode 11b.

The electron transport layer 15 has a function of transporting electrons, which are generated in the photosensitive layer 13, to the conductive support 11. The electron transport layer 15 is formed from an electron transporting material capable of exhibiting the above-described function. The electron transporting material is not particularly limited, and an organic material (organic electron transporting material) is preferable. Examples of the organic electron transporting material include fullerene compounds such as [6,6]-phenyl-C61-butyric acid methyl ester ($PC_{61}BM$), perylene compounds such as perylene tetracarboxylic diimide (PTCDI), low-molecular-weight compounds such as tetracyanoquinodimethane (TCNQ), high-molecular-weight compounds, or the like.

Although not particularly limited, it is preferable that the film thickness of the electron transport layer 15 is 0.001 to 10 $\mu$m, and more preferably 0.01 to 1 $\mu$m.

-Hole transport layer 16-

In the present invention, as in the photoelectric conversion element 10E, the hole transport layer 16 is preferably provided on the surface of the transparent electrode 11b.

The hole transport layer 16 is the same as the hole transport layer 3 to be described later except for a different formation position.

-Photosensitive layer (light absorbing layer) 13-

The photosensitive layer 13 is preferably provided on the surface (including an inner surface of a concave portion in a case where a surface on which the photosensitive layer 13 is provided is uneven) of each of the porous layer 12 (in the photoelectric conversion elements 10A, 10B, and 10F), the blocking layer 14 (in the photoelectric conversion element 10C), the electron transport layer 15 (in the photoelectric conversion element 10D), and the hole transport layer

16 (in the photoelectric conversion element 10E).

In the present invention, the light absorbing agent is contained in the photosensitive layer 13. This light absorbing agent contains at least one kind of each of an organic cation represented by Formula (1) to be described later (hereinafter referred to as organic cation (A1)) and an organic cation represented by Formula (II) (hereinafter referred to as organic cation (A2)), and further contains a cation of a metal atom and an anion. At least a part of the light absorbing agent has the perovskite-type crystal structure.

In addition, the photosensitive layer may have a light absorbing component such as a metal complex dye and an organic dye in addition to the above light absorbing agent.

The photosensitive layer 13 may be a monolayer or a laminated structure of two or more layers. In a case where the photosensitive layer 13 has the laminated layer structure of two or more layers, the laminated layer structure may be a laminated layer structure obtained by laminating layers formed from light absorbing agents different from each other, or a laminated layer structure including an interlayer including a hole transporting material between a photosensitive layer and a photosensitive layer. In a case where the photosensitive layer 13 is the laminated structure of two or more layers, as long as at least one layer has the perovskite compound having at least one kind of each of the organic cation (A1) and the organic cation (A2), the photosensitive layer 13 may have any layer, or may have all layers.

The aspect in which the photosensitive layer 13 is provided on the conductive support 11 is as described above. The photosensitive layer 13 is preferably provided on a surface of each of the layers in order for an excited electron to flow to the conductive support 11 or the second electrode 2. At this time, the photosensitive layer 13 may be provided on the entirety or a part of the surface of each of the layers.

The film thickness of the photosensitive layer 13 is appropriately set in correspondence with an aspect in which the photosensitive layer 13 is provided on the conductive support 11, and is not particularly limited. In general, for example, the film thickness is preferably 0.001 to 100 $\mu$m, more preferably 0.01 to 10 $\mu$m, and still more preferably 0.01 to 5 $\mu$m.

In a case where the porous layer 12 is provided, a total film thickness including the film thickness of the porous layer 12 is preferably 0.01 $\mu$m or greater, more preferably 0.05 $\mu$m or greater, still more preferably 0.1 $\mu$m or greater, and still more preferably 0.3 $\mu$m or greater. In addition, the total film thickness is preferably 100 $\mu$m or less, more preferably 50 $\mu$m or less, and still more preferably 30 $\mu$m or less. The total film thickness may be set to a range in which the above-described values are appropriately combined. Here, as illustrated in Fig. 1, in a case where the photosensitive layer 13 has a thin film shape, the film thickness of the photosensitive layer 13 represents a distance between an interface with the porous layer 12, and an interface with the hole transport layer 3 to be described later along a direction that is perpendicular to the surface of the porous layer 12.

In a case where the photoelectric conversion element 10 includes the porous layer 12 and the hole transport layer 3, a total film thickness of the porous layer 12, the photosensitive layer 13, and the hole transport layer 3 is not particularly limited. For example, the total thickness is preferably 0.01 $\mu$m or greater, more preferably 0.05 $\mu$m or greater, still more preferably 0.1 $\mu$m or greater, and still more preferably 0.3 $\mu$m or greater. In addition, the total film thickness is preferably 200 $\mu$m or less, more preferably 50 $\mu$m or less, even more preferably 30 $\mu$m or less, and particularly preferably 5 $\mu$m or less. The total film thickness may be set to a range in which the above-described values are appropriately combined.

In the present invention, in a case where the photosensitive layer is provided in a thick film shape (in the photosensitive layer 13B and 13C), the light absorbing agent that is included in the photosensitive layer may function as a hole transporting material.

[Light absorbing agent]

In the photoelectric conversion elements of the embodiment of the invention, a light absorbing agent constituting the photosensitive layer has two specific organic cations (A1) and (A2) having different structures from each other, a cation of a metal atom, and an anion, and at least a part of the light absorbing agent has the perovskite-type crystal structure.

In the photoelectric conversion element using a perovskite compound of the related art as a light absorbing agent, the light absorbing agent is likely to be decomposed by moisture existing outside or inside in advance, and the photoelectric conversion efficiency greatly deteriorates particularly under a high-humidity environment. The reason for this is uncertain, but the condition in which migration of an anion constituting the crystal structure in the photosensitive layer to an adjacent layer (for example, the hole transport layer 3, the electron transport layer 4, or the second electrode 2) is likely to occur due to the influence of moisture, and as a result, defects are generated at the interface between the photosensitive layer and the adjacent layer, moisture enters the photosensitive layer, and therefore decomposition of the crystal structure in the photosensitive layer proceeds, is perceived as one of the causes.

In the present invention, the organic cation (A1) having a specific structure which is highly hydrophobic and bulky (hereinafter referred to as "hydrophobic group") is used in the light absorbing agent constituting the photosensitive

layer. It is presumed that this hydrophobic group suppresses the migration of the anion constituting the crystal structure, thereby preventing the migration of the anion to the adjacent layer, and as a result, decomposition of the crystal structure in the photosensitive layer can be effectively suppressed even under a high-humidity environment. In addition, by introducing an acidic group into the hydrophobic group, an action of suppressing decomposition of the crystal structure can be further enhanced. The reason for this is uncertain, but the condition in which movement of anions can be more strictly restricted due to hydrogen-bonding interaction between an acidic group and an anion or between acidic groups in the perovskite-type crystal structure, and the like, is perceived as one of the causes.

Furthermore, in the present invention, two kinds of the organic cations (A1) and (A2) having different hydrophilic and hydrophobic properties and steric bulkiness are coexisted in the light absorbing agent constituting the photosensitive layer. It is presumed that this combination use of these two kinds of the organic cations makes arrangement of the organic cations to be suitable for a surface state of the adjacent layer on the surface of the photosensitive layer possible, and thus contributes to more effective suppression of interface defects. In the present invention, the term "high-humidity" means, for example, an environment in which a relative humidity is 50% or more.

The organic cation (A1) composing the light absorbing agent is represented by Formula (1).

Formula (1) 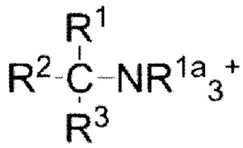

In Formula (1), $R^1$ and $R^2$ represent a group selected from a substituent group Z below, and $R^3$ represents a hydrogen atom or a group selected from the substituent group Z below. Provided that not all $R^1$, $R^2$ and $R^3$ are acidic groups.

The substituent group Z consists of a group represented by Formula (1a) to be described later, an aryl group, a cycloalkyl group, a heteroaryl group, an aliphatic heterocyclic group, an alkenyl group, an alkynyl group, and an acidic group.

The aryl groups which can be taken as $R^1$, $R^2$, and $R^3$ preferably have 6 to 14 carbon atoms. Preferred specific examples of the aryl group include phenyl and naphthyl, and phenyl is more preferable.

The cycloalkyl groups which can be taken as $R^1$, $R^2$, and $R^3$ preferably have 3 to 8 carbon atoms. Preferred specific examples of the cycloalkyl group include cyclopropyl, cyclopentyl, and cyclohexyl.

The heteroaryl groups which can be taken as $R^1$, $R^2$, and $R^3$ include a monocyclic group composed of an aromatic hetero ring, and a group composed of a fused hetero ring obtained through condensing of another ring (for example, an aromatic ring, an aliphatic ring, or a hetero ring) with the aromatic hetero ring.

As the ring-constituting heteroatom that constitutes the aromatic hetero ring, a nitrogen atom, an oxygen atom, or a sulfur atom is preferable. In addition, with regard to the number of ring members of the aromatic hetero ring, a 3- to 8-membered ring is preferable, and a 5- or 6-membered ring is more preferable.

Examples of the 5-membered aromatic hetero ring include a pyrrole ring, an imidazole ring, a pyrazole ring, an oxazole ring, a thiazole ring, a triazole ring, a furan ring, and a thiophene ring. In addition, examples of the fused hetero ring containing a 5-membered aromatic hetero ring include a benzimidazole ring, a benzoxazole ring, a benzothiazole ring, an indoline ring, and an indazole ring.

In addition, examples of the 6-membered aromatic hetero ring include a pyridine ring, a pyrimidine ring, a pyrazine ring, and a triazine ring. In addition, examples of the fused hetero ring containing a 6-membered aromatic hetero ring include a quinoline ring and a quinazoline ring.

The aliphatic heterocyclic groups which can be taken as $R^1$, $R^2$, and $R^3$ include a monocyclic group composed of an aliphatic hetero ring alone, and a group composed of a fused aliphatic hetero ring obtained through condensing of another ring (for example, an aliphatic ring) with the aliphatic hetero ring. As the ring-constituting heteroatom that constitutes the aliphatic hetero ring, a nitrogen atom, an oxygen atom, or a sulfur atom is preferable. In addition, with regard to the number of ring members of the aliphatic hetero ring, three-membered to eight-membered rings are preferable, and a five-membered ring or a six-membered ring is more preferable. The number of carbon atoms of the aliphatic hetero ring is preferably from 0 to 24, more preferably from 1 to 18, even more preferably from 2 to 10, and particularly preferably from 3 to 5.

Specific preferred examples of the aliphatic hetero ring include a pyrrolidine ring, an oxolane ring, a thiolane ring, a piperidine ring, a tetrahydrofuran ring, an oxane ring (tetrahydropyran ring), a thiane ring, a piperazine ring, a morpholine ring, a quinuclidine ring, an azetidine ring, an oxetane ring, an aziridine ring, a dioxane ring, a pentamethylene sulfide ring, γ-butyrolactone, and the like.

The alkenyl groups which can be taken as $R^1$, $R^2$, and $R^3$ include a linear alkenyl group and a branched alkenyl group. The number of carbon atoms of the alkenyl group is preferably from 2 to 18, more preferably from 3 to 10, even more preferably from 4 to 8, and particularly preferably from 5 to 7. Preferred specific examples of the alkenyl group

include vinyl, allyl, butenyl, pentenyl, and hexenyl.

The alkynyl groups which can be taken as $R^1$, $R^2$, and $R^3$ include a linear alkynyl group and a branched alkynyl group. The number of carbon atoms of the alkynyl group is preferably from 2 to 18, more preferably from 3 to 10, even more preferably from 4 to 8, and particularly preferably from 5 to 7. Preferable specific examples of the alkynyl group include ethynyl, propynyl, butynyl, pentynyl, hexynyl, and octynyl.

In regard to the alkenyl groups which can be taken as $R^1$, $R^2$, and $R^3$, the longest carbon chain established as the alkenyl group is estimated in $R^1$, $R^2$, and $R^3$, and the entirety of this longest carbon chain is considered as the alkenyl group. For example, regarding an organic cation a64 exemplified later, the organic cation a64 is a structure having a group represented by "$-CH_2-CH_2-CH(-CH_2-TMS)-CH=CH-CH_2-CH_3$" as $R^1$, $R^2$, or $R^3$, and in this case, the group represented by "$-CH_2-CH_2-CH(-CH_2-TMS)-CH=CH-CH_2-CH_3$" is considered as a branched alkenyl group having 8 carbon atoms, including "$-CH_2-$" in "$(-CH_2-TMS)$" (in this case, "TMS" in "$(-CH_2-TMS)$" is considered as a substituent that the branched alkenyl group has). That is, the organic cation a64 can be considered as a cation in which $R^{1b}$ is a silyl group, n is 4, and one of $R^{1c}$'s is a linear alkenyl group having 4 carbon atoms (1-butenyl group) in the group represented by Formula (1a) to be described later; however, in the present invention, the organic cation a64is is considered as a branched alkenyl group having a silyl group with a substituent of "$-CH_2-CH_2-CH(-CH_2-TMS)-CH=CH-CH_2-CH_3$." This also applies to the alkynyl group.

In the present invention, the acidic group is a group having a dissociative proton. Examples of the acidic group include $-COOH$, $-SO_3H$, $-P(=O)(OH)_2$, $-OP(=O)(OH)_2$, $-P(=O)R^X(OH)$, $-OP(=O)R^X(OH)$, $-P(=O)(OR^Y)(OH)$, $-OP(=O)(OR^Y)(OH)$, $-B(OH)_2$, $-B(OR^Y)(OH)$, $-OB(OH)_2$, $-OB(OR^Y)(OH)$, and $-C_6H_5(OH)$.

$R^X$ represents a substituent, preferably represents an alkyl group (alkyl group preferably having 1 to 25 carbon atoms, more preferably having 1 to 16 carbon atoms), an aryl group (aryl group preferably having 6 to 12 carbon atoms, more preferably having 6 to 10 carbon atoms), a cycloalkyl group (cycloalkyl group preferably having 3 to 7 carbon atoms, more preferably having 3 to 6 carbon atoms), an alkenyl group (alkenyl group preferably having 2 to 16 carbon atoms, more preferably having 3 to 8 carbon atoms), or an alkynyl group (alkynyl group preferably having 2 to 16 carbon atoms, more preferably having 3 to 8 carbon atoms).

In addition, $R^Y$ represents a substituent, preferably represents an alkyl group (alkyl group preferably having 1 to 25 carbon atoms, more preferably having 1 to 16 carbon atoms), or an aryl group (aryl group preferably having 6 to 12 carbon atoms, more preferably having 6 to 10 carbon atoms).

The acidic group in the present invention is preferably a group selected from $-COOH$, $-SO_3H$, $-P(=O)(OH)_2$, and $-B(OH)_2$.

The acidic group may take a form dissociated by releasing protons, or may be in salt form.

The aryl group, the cycloalkyl group, the heteroaryl group, the aliphatic heterocyclic group, the alkenyl group, and the alkynyl group composing the substituent group Z may have a substituent. The substituent is not particularly limited, and examples thereof include the alkyl group, the cycloalkyl group, the alkenyl group, the alkynyl group, the aryl group, the heteroaryl group, and the aliphatic heterocyclic group. Preferred aspects of the alkyl group, the cycloalkyl group, the alkenyl group, the alkynyl group, the aryl group, the heteroaryl group, and the aliphatic heterocyclic group are respectively the same as the preferred aspects of the alkyl group, the cycloalkyl group, the alkenyl group, the alkynyl group, the aryl group, the heteroaryl group, and the aliphatic heterocyclic group in $R^{1a}$ to be described later. Provided that the alkenyl group and the alkynyl group do not have the alkyl group as the substituent.

In Formula (1), $R^{1a}$ represents a hydrogen atom or a substituent. The substituent is preferably a group selected from the alkyl group, the cycloalkyl group, the alkenyl group, the alkynyl group, the aryl group, the heteroaryl group, and the aliphatic heterocyclic group.

The alkyl group which can be taken as $R^{1a}$ includes a linear alkyl group and a branched alkyl group. The number of carbon atoms of the alkyl group is preferably from 1 to 30, more preferably from 1 to 18, even more preferably from 1 to 12, still even more preferably from 1 to 6, and particularly preferably from 1 to 3. Preferred specific examples of the alkyl group include methyl, ethyl, propyl, isopropyl, n-butyl, t-butyl, pentyl, hexyl, decyl, and octadecyl.

The alkenyl group which can be taken as $R^{1a}$ includes a linear alkenyl group and a branched alkenyl group. The number of carbon atoms of the alkenyl group is preferably from 2 to 18, more preferably from 2 to 7, and even more preferably from 2 to 5. Preferred specific examples of the alkenyl group include vinyl, allyl, butenyl, pentenyl, and hexenyl.

The alkynyl group which can be taken as $R^{1a}$ includes a linear alkynyl group and a branched alkynyl group. The number of carbon atoms of the alkynyl group is preferably from 2 to 18, more preferably from 2 to 7, and even more preferably from 2 to 5. Preferable specific examples of the alkynyl group include ethynyl, propynyl, butynyl, pentynyl, hexynyl, and octynyl.

The cycloalkyl group, the aryl group, the heteroaryl group, and the aliphatic heterocyclic group which can be taken as $R^{1a}$ are respectively the same as the cycloalkyl group, the aryl group, the heteroaryl group, and the aliphatic heterocyclic group which can be taken as $R^1$, $R^2$, and $R^3$, and preferred range thereof is also the same. Two $R^{1a}$'s adjacent to each other which are linked to the N atom may form a ring by being linked to each other. In this case, the formed ring may have a heteroatom as a ring-constituting atom.

$R^{1a}$ is preferably a hydrogen atom or an alkyl group and is more preferably a hydrogen atom.

The substituent group Z includes the group represented by Formula (1a).

$$\text{Formula (1a)} \qquad R^{1b}-(CR^{1c}_2)_n-*$$

In Formula (1a), $R^{1b}$ represents a methyl group, a silyl group, a cycloalkyl group, an aryl group, a heteroaryl group, an aliphatic heterocyclic group, a halogen atom, or an acidic group. $R^{1c}$ represents a hydrogen atom or represents a substituent other than an alkenyl group and other than an alkynyl group. n represents integer of 3 to 15. n is more preferably an integer of 3 to 12, even more preferably an integer of 3 to 10, and still even more preferably an integer of 4 to 8.

* represents a binding site with a carbon atom represented by Formula (1).

In Formula (1a), a silyl group which can be taken as $R^{1b}$ is not particularly limited, and examples thereof include a silyl group represented by Formula (Is).

$$\text{Formula (1s)} \qquad -Si(R^{1s})_3$$

Examples of $R^{1s}$ include a hydrogen atom, and a group selected from the alkyl group, the cycloalkyl group, the alkenyl group, the alkynyl group, the aryl group, the heteroaryl group, and the aliphatic heterocyclic group, and a preferred range thereof is the same as the preferred range of each corresponding group in $R^{1a}$. As $R^{1s}$, a hydrogen atom, an alkyl group, a cycloalkyl group, an alkenyl group are preferable, and an alkyl group is particularly preferable.

Preferred specific examples of the silyl group include a trimethylsilyl group, a triethylsilyl group, a triisopropylsilyl group, a t-butyldimethylsilyl group, a tributylsilyl group, a t-butyldiphenylsilyl group, and a dimethylphenyl group.

In Formula (1a), a preferred range of the cycloalkyl group, the aryl group, the heteroaryl group, and the aliphatic heterocyclic group which can be taken as $R^{1b}$, and the acidic group is the same as the preferred range of each corresponding group in the substituent group Z.

In Formula (1a), examples of the halogen atom which can be taken as $R^{1b}$ include a fluorine atom, a chlorine atom, a bromine atom, or an iodine atom, and the fluorine atom is preferable.

In Formula (1a), the substituent which can be taken as $R^{1c}$ is not particularly limited as long as the substituent is the substituent excluding the alkenyl group and the alkynyl group. The substituent which can be taken as $R^{1c}$ is preferably the alkyl group, the cycloalkyl group, the aryl group, the heteroaryl group, and the aliphatic heterocyclic group, or the acidic group.

The alkyl group which can be taken as $R^{1c}$ is the same as the alkyl group which can be taken as the above-described $R^{1a}$, and a preferred range thereof is also the same.

The cycloalkyl group, the aryl group, the heteroaryl group, and the aliphatic heterocyclic group which can be taken as $R^{1c}$, and the acidic group are respectively the same as the cycloalkyl group, the aryl group, the heteroaryl group, and the aliphatic heterocyclic group which can be taken as $R^1$, $R^2$, and $R^3$ in Formula (1), and the acidic group, and preferred range thereof is also the same.

In Formula (1a), n is preferably from 3 to 8 and more preferably from 3 to 5.

It is preferable that, in the organic cation represented by Formula (1), $R^1$ and $R^2$ represent a group selected from a group represented by Formula (1a), an aryl group, a cycloalkyl group, a heteroaryl group, an aliphatic heterocyclic group, an alkenyl group, and an alkynyl group, and $R^3$ represents a hydrogen atom or a group selected from the substituent group Z. By adopting such a structure, it is possible to further suppress the decomposition of the crystal structure constituting the photosensitive layer of the photoelectric conversion element, and it is possible to further suppress a deterioration in the photoelectric conversion efficiency over time.

From the viewpoint of suppressing the decomposition of the crystal structure constituting the photosensitive layer, in the compound represented by Formula (1), all of $R^1$, $R^2$, and $R^3$ more preferably represent a group selected from the group represented by Formula (1a), the aryl group, the cycloalkyl group, the heteroaryl group, the aliphatic heterocyclic group, the alkenyl group, and the alkynyl group. In this case, $R^1$ and $R^2$ even more preferably represent a group selected from the group represented by Formula (1a), the alkenyl group, and the alkynyl group.

In the compound represented by Formula (1), it is more preferable that $R^1$ and $R^2$ represent the group represented by Formula (1a), $R^{1b}$ in $R^1$ represents a methyl group, a silyl group, a cycloalkyl group, an aryl group, or a halogen atom, and $R^{1b}$ in $R^2$ represents an acidic group, and it is even more preferable that $R^{1b}$ in $R^1$ represents a methyl group, a silyl group, or a cycloalkyl group, and $R^{1b}$ in $R^2$ represents an acidic group. In this case, $R^3$ is preferably the group represented by Formula (1a).

Specific examples of the organic cation represented by Formula (1) are shown below, but the present invention

is not limited thereto.

In the following specific examples, TMS represents a trimethylsilyl group.

a1
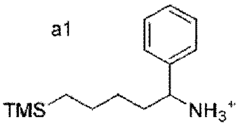
a2
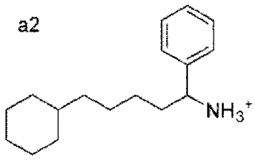
a3
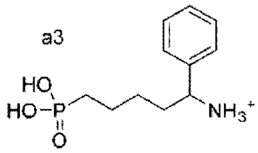
a4
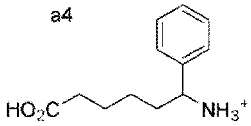

a5
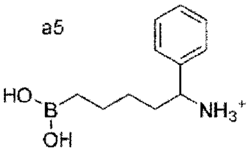
a6
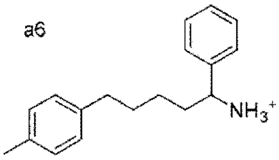
a7
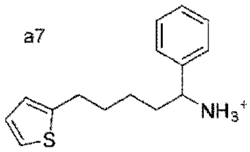
a8
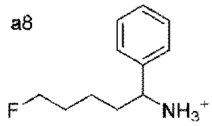

a9
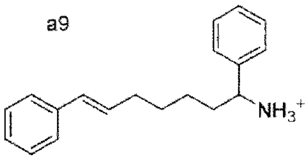
a10
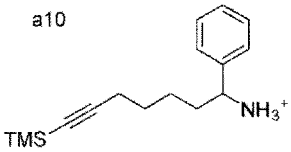

a11
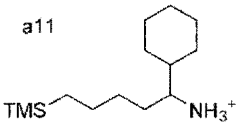
a12
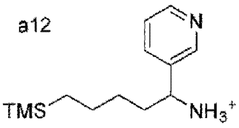
a13
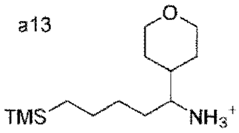
a14
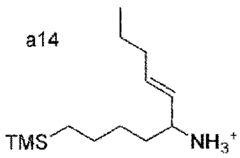

a15
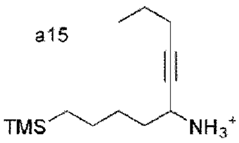
a16
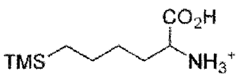
a17
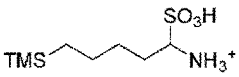

a18
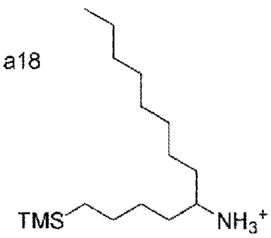
a19
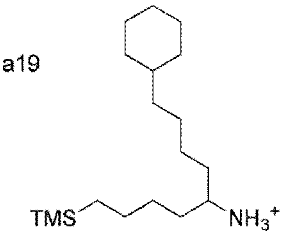
a20
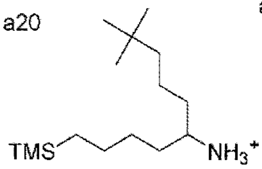
a21
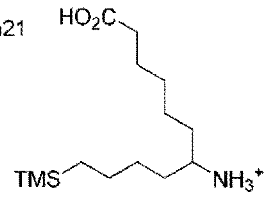

a22
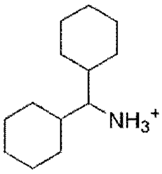
a23
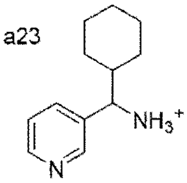
a24
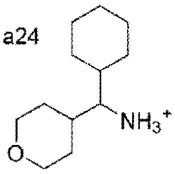
a25
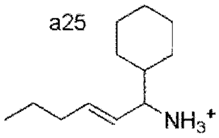

a26
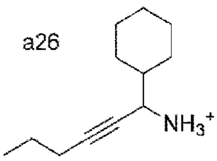
a27
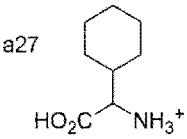
a28
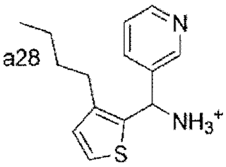
a29
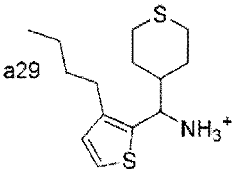

a30 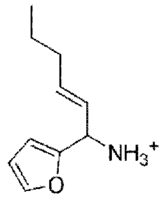 a31 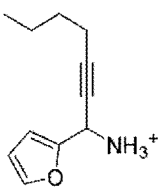

a32 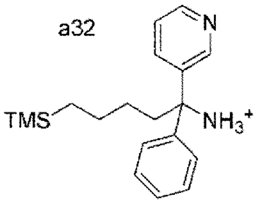 a33 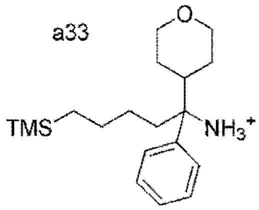 a34 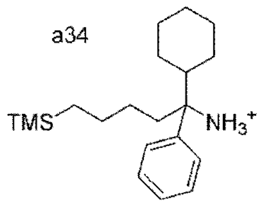 a35 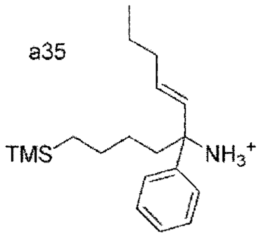

a36 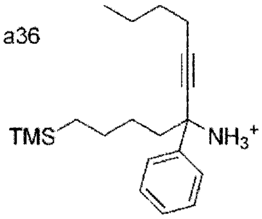

a37 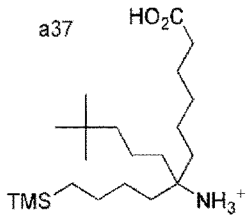 a38 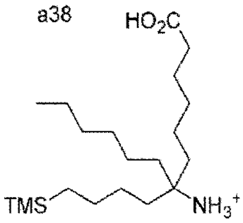 a39 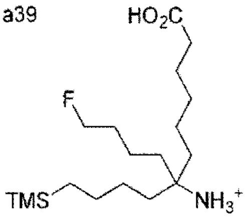 a40 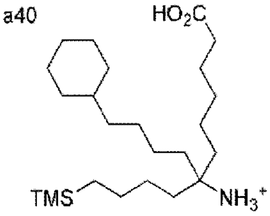

a41 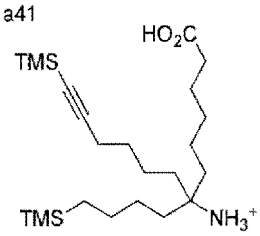 a42 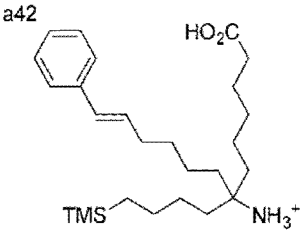

a43 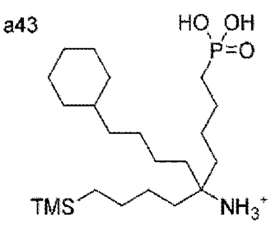 a44 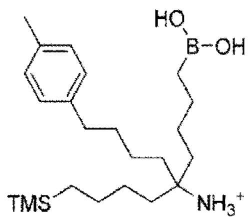 a45 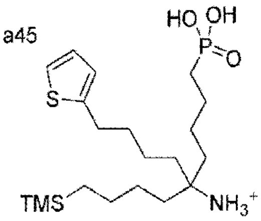

a46
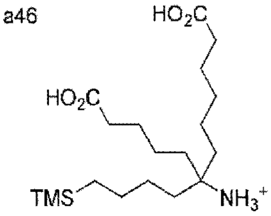
a47
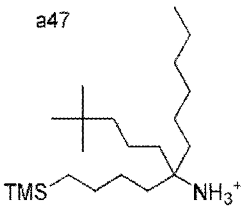
a48
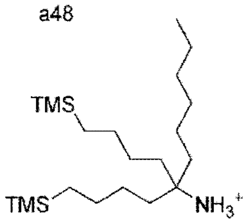

a49
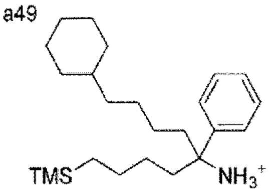
a50
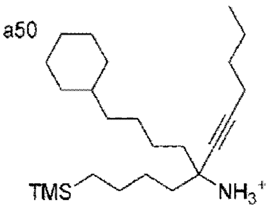
a51
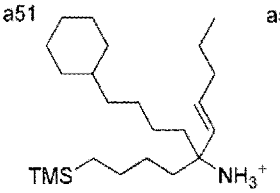
a52
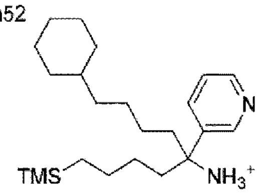

a53
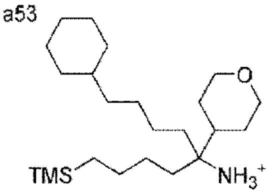

a54
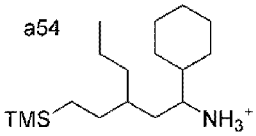
a55
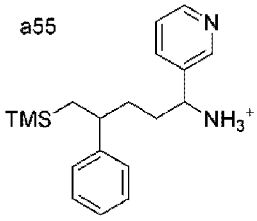
a56
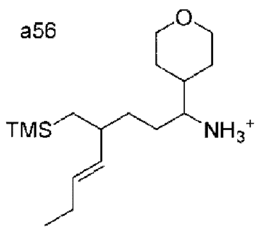
a57
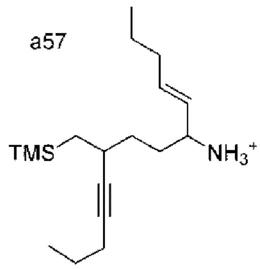

a58
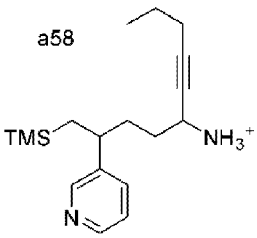
a59
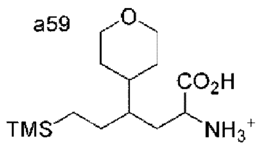
a60
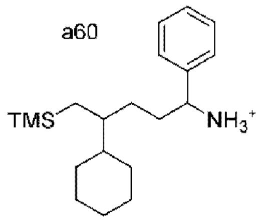

a61
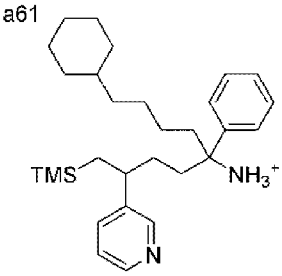
a62
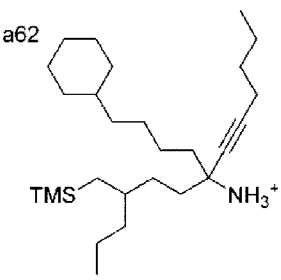
a63
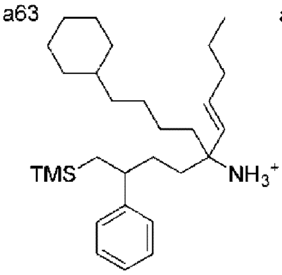
a64
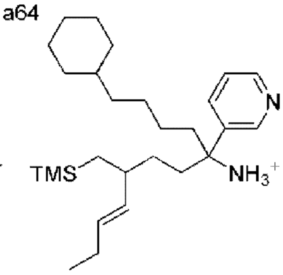

a65

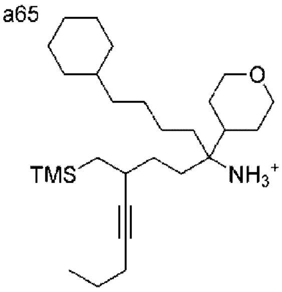

a66

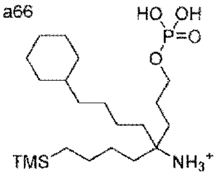

a67

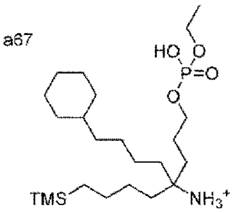

a68

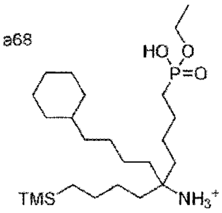

a69

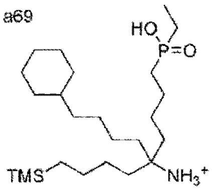

a70

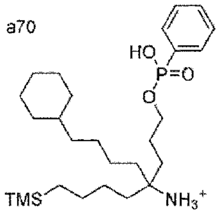

a71

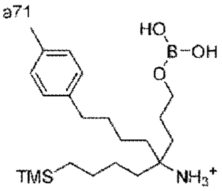

a72

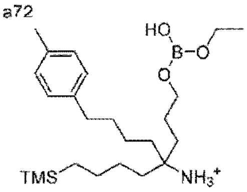

a73

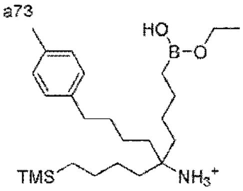

The organic cation (A2) composing the light absorbing agent is represented by Formula (2).

Formula (2) $R^4\text{-}NR^{2a}{}_3{}^+$

In Formula (2), $R^4$ represents a methyl group, an ethyl group, or a group represented by Formula (2a).

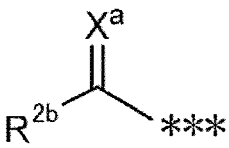

Formula (2a)

In the group represented by Formula (2a), $X^a$ represents $NR^{2c}$, an oxygen atom, or a sulfur atom, and $NR^{2c}$ is preferable. $R^{2c}$ represents a hydrogen atom or a substituent. A substituent which can be taken as $R^{2c}$ is not particularly limited, but the alkyl group, the cycloalkyl group, the alkenyl group, the alkynyl group, the aryl group, the heteroaryl group, or the aliphatic heterocyclic group is preferable. $R^{2c}$ is preferably a hydrogen atom.

$R^{2b}$ represents a hydrogen atom or a substituent, and is preferably a hydrogen atom. A substituent which can be taken as $R^{2b}$ is not particularly limited, and examples thereof include the amino group, the alkyl group, the cycloalkyl group, the alkenyl group, the alkynyl group, the aryl group, the heteroaryl group, or the aliphatic heterocyclic group.

The alkyl groups which can be taken as $R^{2b}$ and $R^{2c}$ are the same as the alkyl group which can be taken as the above-described $R^{1a}$, and a preferred range thereof is also the same.

The cycloalkyl group, the alkenyl group, the alkynyl group, the aryl group, the heteroaryl group, and the aliphatic heterocyclic group which can be taken as $R^{2b}$ and $R^{2c}$ are respectively the same as the cycloalkyl group, the alkenyl group, the alkynyl group, the aryl group, the heteroaryl group, and the aliphatic heterocyclic group which can be taken as $R^1$, $R^2$, and $R^3$ in Formula (1), and preferred range thereof is also the same.

Examples of the group represented by Formula (2a) include a (thio)acyl group, a (thio)carbamoyl group, an imidoyl group, and an amidino group.

Examples of the (thio)acyl group include an acyl group and a thioacyl group. The acyl group is preferably an acyl group having a total of 1 to 7 carbon atoms, and examples thereof include formyl, acetyl ($CH_3C(=O)$-), propionyl,

hexanoyl, and the like. The thioacyl group is preferably a thioacyl group having a total of 1 to 7 carbon atoms, and examples thereof include thioformyl, thioacetyl ($CH_3C(=S)-$), thiopropionyl, and the like.

Examples of the (thio)carbamoyl group include a carbamoyl group ($H_2NC(=O)-$) and a thiocarbamoyl group ($H_2NC(=S)-$).

The imidoyl group is a group represented by $R^{2b}-C(=NR^{2c})-$. It is preferable that $R^{2b}$ and $R^{2c}$ are respectively a hydrogen atom or an alkyl group, and this alkyl group is the same as the alkyl group which can be taken as the above-described $R^{1a}$. Examples of the imidoyl group include formimidoyl ($HC(=NH)-$), acetimidoyl ($CH_3C(=NH)-$), propionimidoyl ($CH_3CH_2C(=NH)-$), and the like. Among these, formimidoyl is preferable.

The amidino group as the group represented by Formula (2a) has a structure ($-C(=NH)NH_2$) in which $R^{2b}$ of the imidoyl group is an amino group and $R^{2c}$ is a hydrogen atom.

*** represents a binding site with a nitrogen atom in Formula (2).

In Formula (2), $R^{2a}$ represents a hydrogen atom, an alkyl group, a cycloalkyl group, an alkenyl group, an alkynyl group, an aryl group, a heteroaryl group, or an aliphatic heterocyclic group.

The alkyl group, the cycloalkyl group, the alkenyl group, the alkynyl group, the aryl group, the heteroaryl group, or the aliphatic heterocyclic group which can be taken as $R^{2a}$ are respectively the same as the alkyl group, the cycloalkyl group, the alkenyl group, the alkynyl group, the aryl group, the heteroaryl group, or the aliphatic heterocyclic group which can be taken as the above-described $R^{1a}$, and preferred range thereof is also the same.

Two $R^{2a}$'s adjacent to each other which are linked to a nitrogen atom may form a ring by being linked to each other. In this case, the formed ring may have a heteroatom as a ring-constituting atom.

$R^{2a}$ is preferably a hydrogen atom or an alkyl group and is more preferably a hydrogen atom.

In Formula (2), in a case where all three $R^{2a}$ are hydrogen atoms, the compound represented by Formula (2) has a structure in which $R^4$ and $NH_3$ in Formula (2) are bonded to have an organic ammonium cation. In a case where the organic ammonium cation can employ a resonance structure, a cation having the resonance structure can be further contained in addition to the organic ammonium cation. For example, in a case where $X^a$ is NH ($R^{2c}$ is a hydrogen atom) in the group that can be represented by Formula (2a), as the structure of the moiety represented by $R^4-NR^{2a}{}_3$ in Formula (2), in addition to the organic ammonium cation structure, a structure of an organic amidinium cation which is one of resonance structures of this organic ammonium cation can also be adopted. Examples of the organic amidinium cation include a cation represented by Formula ($A^{am}$). In the present specification, a form of a resonance structure such as a cation represented by Formula ($A^{am}$) is also included in the group represented by Formula (2a).

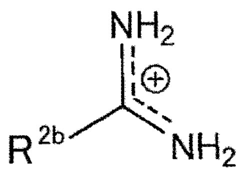

Formula ($A^{am}$)

In the perovskite compound constituting the light absorbing agent in the present invention, a molar ratio of a content a1 of the organic cation represented by Formula (1) to a content a2 of the organic cation represented by Formula (2) preferably satisfies Formula (r1-1), and more preferably satisfies Formula (r1-2), even more preferably satisfies Formula (r1-3), and particularly preferably satisfies Formula (r1-4).

Formula (r1-1) $0.001 \leq a2/a1 \leq 9999$

Formula (r1-2) $4 \leq a2/a1 \leq 4999$

Formula (r1-3) $9 \leq a2/a1 \leq 1999$

Formula (r1-4) $9 \leq a2/a1 \leq 999$

In the perovskite compound constituting the light absorbing agent in the present invention, the cation of the metal atom is preferably a cation of a metal atom other than the elements of Group 1 in the periodic table. Examples of the metal atom include metal atoms such as calcium (Ca), strontium (Sr), cadmium (Cd), copper (Cu), nickel (Ni), manganese (Mn), iron (Fe), cobalt (Co), palladium (Pd), germanium (Ge), tin (Sn), lead (Pb), ytterbium (Yb), europium (Eu), indium (In), titanium (Ti), bismuth (Bi), and thallium (Tl). Among them, a Pb atom, a Cu atom, a Ge atom, or a Sn atom is particularly preferable.

The cation of the metal atom may be one kind of cation or may be two or more kinds of cations. In a case of two or more kinds of cations, a proportion (ratio of contents) therebetween is not particularly limited.
In the perovskite compound that is used in the present invention, the anion represents an anion of an anionic atom or atomic group. Preferred examples of the anion include anions of halogen atoms, and anions of individual atomic groups of $NCS^-$, $NCO^-$, $OH^-$, $NO_3^-$, $CH_3COO^-$, or $HCOO^-$. Among these, the anions of halogen atoms are more preferable. Examples of the halogen atoms include a fluorine atom, a chlorine atom, a bromine atom, an iodine atom, and the like.
The X anion may be an anion of one kind of anionic atom or atomic group, or anions of two or more kinds of anionic atoms or atomic groups. In a case where the anion X is an anion of one kind of anionic atom or atomic group, an anion of an iodine atom is preferable. On the other hand, in a case where the anion X includes anions of two or more kinds of anionic atoms or atomic groups, anions of two kinds of halogen atoms, particularly, an anion of a chlorine atom and an anion of an iodine atom are preferable. The proportion (ratio of contents) between two or more kinds of anions is not particularly limited.
In the photoelectric conversion element of the embodiment of the invention, the perovskite compound constituting at least a part of the light absorbing agent is represented by Formula (i), for example.

Formula (i) $A_aM_mX_x$

In Formula, A represents a cationic organic group. M represents a metal atom. X represents an anionic atom or an anionic atomic group.
a represents 1 or 2, m represents 1, and a, m, and x satisfy a relationship of $a+2m = x$.

In Formula (i), the cationic organic group A is present as the organic cation in the perovskite-type crystal structure. That is, the cationic organic group A contains one or more kinds of the cationic organic group which is the organic cation (A1), and one or more kinds of the cationic organic group which is the organic cation (A2).
The metal atom M is a metal atom that is present as the above-described cation of the metal atom in the perovskite-type crystal structure.
The anionic atom or an anionic atomic group X is present as the anion in the perovskite-type crystal structure.
The perovskite compound represented by Formula (i) is a perovskite compound represented by the following Formula (i-1) in a case where a is 1, or a perovskite compound represented by the following Formula (i-2) in a case where a is 2.

Formula (i-1) $AMX_3$

Formula (i-2) $A_2MX_4$

The crystal structure of the perovskite compound may further contain organic cations other than the cations of the elements of Group 1 in the periodic table, and the organic cations (A1) and (A2).
The cation of the element of Group 1 in the periodic table is not particularly limited, and examples thereof include cations ($Li^+$, $Na^+$, $K^+$, and $Cs^+$) of individual elements of lithium (Li), sodium (Na), potassium (K), and cesium (Cs), and the cation ($Cs^+$) of cesium is more preferable.
The organic cations other than the organic cation (A1) and the organic cation (A2) are not particularly limited as long as organic cations can be present as the cation in the perovskite-type crystal structure.
In the perovskite compound used in the present invention, a ratio of a total molar amount of the organic cation (A1), the organic cation (A2), and the cation of the metal atom M with respect to a total molar amount of the cations constituting the perovskite-type crystal structure is from 90% to 100% by mol, and more preferably from 95% to 100% by mol.
In addition, in the perovskite compound used in the present invention, a ratio of a total molar amount of the anion of the halogen atom with respect to a total molar amount of the anions constituting the perovskite-type crystal structure is preferably from 90% to 100% by mol, more preferably from 95% to 100% by mol, and even more preferably from 98% to 100% by mol.
In general, the perovskite compound can be synthesized from a compound represented by the following Formula (ii) and a compound represented by the following Formula (iii).

Formula (ii) AX

Formula (iii) $MX_2$

A, M, and X in Formulas (ii) and (iii) are respectively the same as A, M, and X in Formula (i).

Examples of the method for synthesizing the perovskite compound include Science, 2012, vol. 338, p. 643-647; Angew. Chem. Int. Ed. 2014, 53, p. 11232-11235; and Science, 2014, vol. 345, p.295. Examples thereof further include Akihiro Kojima, Kenjiro Teshima, Yasuo Shirai, and Tsutomu Miyasaka, "Organometal Halide Perovskites as Visible-Light Sensitizers for Photovoltaic Cells", J. Am. Chem. Soc., 2009, 131(17), p. 6050 to 6051.

The amount of the light absorbing agent used is preferably set to an amount capable of covering at least a part of the surface of the first electrode 1, and more preferably an amount capable of covering the entirety of the surface.

The amount of the light absorbing agent contained in the photosensitive layer 13 is typically 1% to 100% by mass.

<Hole transport layer 3>

In the photoelectric conversion element of the embodiment of the invention, as in the photoelectric conversion elements 10A to 10D, an aspect in which the hole transport layer 3 is provided between the first electrode 1 and the second electrode 2 is also preferable. In the aspect, it is preferable that the hole transport layer 3 is in contact with (laminated on) the photosensitive layer 13. The hole transport layer 3 is preferably provided between the photosensitive layer 13 of the first electrode 1 and the second electrode 2.

The hole transport layer 3 includes a function of supplementing electrons to an oxidized substance of the light absorbing agent, and is preferably a solid-shaped layer (solid hole transport layer).

A hole transporting material forming the hole transport layer 3 may be a liquid material or a solid material without any limitation. Examples of the hole transporting material include inorganic materials such as CuI and CuNCS, organic hole transporting materials described in paragraphs 0209 to 0212 of JP2001-291534A, and the like. Preferred examples of the organic hole transporting material include conductive polymers such as polythiophene, polyaniline, polypyrrole, and polysilane, spiro compounds in which two rings share a central atom such as C or Si having a tetrahedral structure, aromatic amine compounds such as triarylamine, triphenylene compounds, nitrogen-containing heterocyclic compounds, and liquid-crystalline cyano compounds.

As the hole transporting material, an organic hole transporting material which can be applied in a solution state and then has a solid shape is preferable, and specific examples thereof include 2,2',7,7'-tetrakis-(N,N-di-p-methoxyphenylamino)-9,9'-spirobifluorene (also referred to as spiro-MeOTAD), poly(3-hexylthiophene-2,5-diyl), 4-(diethylamino)benzaldehyde diphenylhydrazone, polyethylene dioxythiophene (PEDOT), and the like.

Although not particularly limited, the film thickness of the hole transport layer 3 is preferably 50 μm or less, more preferably 1 nm to 10 μm, still more preferably 5 nm to 5 μm, and still more preferably 10 nm to 1 μm. In addition, the film thickness of the hole transport layer 3 corresponds to an average distance between the second electrode 2 and the surface of the photosensitive layer 13, and can be measured by observing a cross-section of the photoelectric conversion element by using a scanning electron microscope (SEM) and the like.

<Electron transport layer 4>

In the photoelectric conversion element of the embodiment of the invention, as in the photoelectric conversion element 10E, an aspect in which the electron transport layer 4 is provided between the first electrode 1 and the second electrode 2 is also preferable. In the aspect, it is preferable that the electron transport layer 4 is in contact with (laminated on) the photosensitive layer 13.

The electron transport layer 4 is the same as the electron transport layer 15, except that a destination of electrons is the second electrode and positions where the electrons are formed are different.

<Second electrode 2>

The second electrode 2 functions as a positive electrode in a solar cell. The second electrode 2 is not particularly limited as long as the second electrode 2 has conductivity. Typically, the second electrode 2 can be configured to have the same configuration as that of the conductive support 11. In a case where sufficient strength is maintained, the support 11a is not necessary.

As a structure of the second electrode 2, a structure having a high current-collection effect is preferable. At least one of the conductive support 11 or the second electrode 2 needs to be substantially transparent so that light reaches the photosensitive layer 13. In the solar cell of the embodiment of the invention, it is preferable that the conductive support 11 is transparent and solar light and the like is incident from the support 11a side. In this case, it is more preferable that the second electrode 2 has a light-reflecting property.

Examples of a material used to form the second electrode 2 include metals such as platinum (Pt), gold (Au), nickel (Ni), copper (Cu), silver (Ag), indium (In), ruthenium (Ru), palladium (Pd), rhodium (Rh), iridium (Ir), osmium (Os), and aluminum (A1), the above-described conductive metal oxides, carbon materials, conductive polymers, and the like.

The carbon materials may be conductive materials formed through bonding of carbon atoms, and examples thereof include fullerene, a carbon nanotube, graphite, graphene, and the like.

As the second electrode 2, a thin film (including a thin film obtained through vapor deposition) of a metal or a conductive metal oxide, or a glass substrate or a plastic substrate which has the thin film is preferable. As the glass substrate or the plastic substrate, glass including a gold or platinum thin film or glass on which platinum is vapor-deposited is preferable.

The film thickness of the second electrode 2 is not particularly limited, and is preferably 0.01 to 100 μm, more preferably 0.01 to 10 μm, and still more preferably 0.01 to 1 μm.

<Other configurations>

In the present invention, a spacer or a separator can also be used instead of the blocking layer 14 and the like or in combination with the blocking layer 14 and the like so as to prevent the first electrode 1 and the second electrode 2 from coming into contact with each other.

In addition, a hole blocking layer may be provided between the second electrode 2 and the hole transport layer 3.

[Solar cell]

The solar cell of the embodiment of the invention is constituted by using the photoelectric conversion element of the embodiment of the invention. For example, as illustrated in Figs. 1 to 6, the photoelectric conversion element 10 having a configuration, which is allowed to operate by the external circuit 6, can be used as the solar cell. As the external circuit 6 that is connected to the first electrode 1 (the conductive support 11) and the second electrode 2, a known circuit can be used without particular limitation.

For example, the present invention is applicable to individual solar cells described in Science, 2012, vol. 338, p. 643-647; Angew. Chem. Int. Ed. 2014, 53, p. 11232-11235; and Science, 2014, vol. 345, p.295; J. Am. Chem. Soc., 2009, 131(17), p. 6050 to 6051; and Science, 338, p. 643(2012).

It is preferable that a lateral surface of the solar cell of the embodiment of the invention is sealed with a polymer, an adhesive, and the like so as to prevent deterioration, evaporation, and the like in constituent substances.

[Method for manufacturing photoelectric conversion element and solar cell]

Other than formation of the photosensitive layer, the photoelectric conversion element and the solar cell of the embodiment of the invention can be manufactured in accordance with a known method, for example, a method described in Science, 2012, vol. 338, p. 643-647; Angew. Chem. Int. Ed. 2014, 53, p. 11232-11235; and Science, 2014, vol. 345, p.295; J. Am. Chem. Soc., 2009, 131(17), p. 6050 to 6051; Science, 338, p. 643(2012); and the like.

Hereinafter, the method of manufacturing the photoelectric conversion element and the solar cell of the embodiment of the invention will be described in brief.

In the photoelectric conversion element of the embodiment of the invention, first, at least one of the blocking layer 14, the porous layer 12, the electron transport layer 15, or the hole transport layer 16 is formed on a surface of the conductive support 11 according to the purpose.

For example, the blocking layer 14 can be formed by a method in which a dispersion, which contains the insulating substance or a precursor compound thereof, and the like, is applied to the surface of the conductive support 11, and the dispersion is baked; a spray pyrolysis method; and the like.

A material that forms the porous layer 12 is preferably used as fine particles, and more preferably a dispersion that contains the fine particles.

A method of forming the porous layer 12 is not particularly limited, and examples thereof include a wet-type method, a dry-type method, and other methods (for example, a method described in Chemical Review, Vol. 110, p. 6595 (published on 2010)). In these methods, it is preferable that the dispersion (paste) is applied to the surface of the conductive support 11 or the surface of the blocking layer 14 and then the dispersion is baked at a temperature 100°C to 800°C for ten minutes to ten hours, for example, in the air. According to this, it is possible to bring the fine particles into close contact with each other.

In a case where baking is performed a plurality of times, a temperature in baking except final baking (a baking temperature except for a final baking temperature) is preferably set to be lower than the temperature in the final baking (the final baking temperature). For example, in a case where titanium oxide paste is used, the baking temperature except for the final baking temperature can be set in a range of 50°C to 300°C. In addition, the final baking temperature can be set in a range of 100°C to 600°C to be higher than the baking temperature except for the final baking temperature. In a case where a glass support is used as the support 11a, the baking temperature is preferably 60°C to 500°C.

The amount of a porous material applied to form the porous layer 12 is appropriately set in correspondence

with the film thickness of the porous layer 12, the number of times of coating, and the like, and there is no particular limitation thereto. For example, the amount of the porous material applied per 1 $m^2$ of a surface area of the conductive support 11 is preferably 0.5 to 500 g, and more preferably 5 to 100 g.

In a case where the electron transport layer 15 or the hole transport layer 16 is provided, the layer can be formed in the same manner as in the hole transport layer 3 or the electron transport layer 4 to be described below.

Subsequently, the photosensitive layer 13 is provided.

In the formation of the photosensitive layer 13, a compound capable of synthesizing the perovskite compound is used.

Examples of the compound capable of synthesizing the perovskite compound include the compound AX represented by Formula (ii) and the compound $MX_2$ represented by Formula (iii). Each of these compounds may be used alone or may be used as a composition (including a form of solution, suspension, paste, and the like).

In the present invention, the photosensitive layer 13 can be formed using a light absorbing agent composition containing all the compounds. In addition, the photosensitive layer 13 can be formed using a composition containing a compound represented by Formula (1b) and a compound represented by Formula (2b) (hereinafter, this composition will be referred to as "organic ammonium salt composition." This organic ammonium salt composition preferably has a form of solution, suspension, paste, and the like), and the compound $MX_2$.

In the present invention, as the compound AX, a compound $C(R^1)(R^2)(R^3)\text{-}N(R^{1a})_3X$ and a compound $R^4\text{-}N(R^{2a})_3X$ are preferably used. $R^1$, $R^2$, $R^3$, $R^4$, $R^{1a}$, and $R^{2a}$ each have the same definition as $R^1$, $R^2$, $R^3$, $R^4$, $R^{1a}$, and $R^{2a}$ in Formulas (1) and (2), and the preferable aspect thereof is also the same. In the compounds, X is preferably a halogen atom, and in this case, both compounds are the compounds represented by Formulas (1b) and (2b), respectively, and preferable aspects thereof are also the same.

Formula (1b) 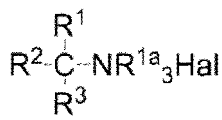

Formula (2b) $R^4\text{-}N(R^{2a})_3Hal$

In the formula, $R^1$, $R^2$, $R^3$, $R^4$, $R^{1a}$, and $R^{2a}$ each have the same definition as $R^1$, $R^2$, $R^3$, $R^4$, $R^{1a}$, and $R^{2a}$ in Formulas (1) and (2), and the preferable aspect thereof is also the same. Hal represents a halogen atom, and preferably represents an iodine atom, a chlorine atom, or a bromine atom.

The light absorbing agent composition contains the compound AX and the compound $MX_2$.

This light absorbing agent composition can be prepared by mixing the compound AX and the compound $MX_2$ at a predetermined molar ratio and then heating the resultant mixture. The formation liquid (light absorbing agent solution) is typically a solution, but may be a suspension. Heating conditions are not particularly limited. For example, a heating temperature is preferably from 30°C to 200°C, and more preferably from 70°C to 150°C. A heating time is preferably from 0.5 to 100 hours, and more preferably from 1 to 3 hours. As a solvent or a dispersion medium, the following solvent or dispersion medium can be used.

It is sufficient as long as the ammonium salt composition contains each one kind of the compound represented by Formula (1b) and the compound represented by Formula (2b), and the ammonium salt composition may contain two or more kinds thereof.

The ammonium salt composition may contain the compound $MX_2$, and preferably a halogenated metal (in a case where X is a halogen atom). In this case, the ammonium salt composition is a preferred aspect of the light absorbing agent composition.

Examples of the halogenated metal include a halide of the metal atom M (that is, the compound represented by $M(Hal)_2$, Hal representing a halogen atom) that the above-described perovskite compound used in the present invention has. The halogenated metal is preferably at least one kind selected from a halide of Pb or a halide of Sn, more preferably at least one kind selected from an iodide of Pb, a chloride of Pb, an iodide of Sn, or an iodide of chlorine, and particularly preferably at least one kind selected from $PbI_2$ or $SnI_2$.

In a case where the ammonium salt composition does not contain the compound $MX_2$, a metal salt composition compound containing the $MX_2$, preferably the halogenated metal is used in addition to the ammonium salt composition. This metal salt composition is preferably a solution, and is prepared by heating as necessary. Heating conditions are not particularly limited. For example, a heating temperature is preferably from 30°C to 200°C, and more preferably from 50°C to 120°C. A heating time is preferably from 5 minutes to 48 hours, and more preferably from 0.5 to 24 hours.

All of the light absorbing agent composition, the ammonium salt composition, and the metal salt composition may be solid (powder, granular, and the like), but are preferably solutions. In a case where these compositions are solutions, an organic solvent is preferable as a medium to be used. The organic solvent will be described later.

All of the compositions may contain other components in addition to the above-described compounds. Examples of the other components include halides of the elements of Group 1 in the periodic table, and the like.

All of the above compositions can be suitably used as a supply source of the compound $MX_2$ and/or the compound AX in the formation of the photosensitive layer in the photoelectric conversion element of the embodiment of the invention. In a case where these compositions are in a form of powder, granule, or the like, the compositions can be used by dissolving the compositions in a solvent to prepare a solution with an appropriate concentration, and then, as necessary, performing filtering, purifying, and the like. In addition, in a case where the compositions are solutions, the compositions can be used as they are after the compositions are subjected to concentration, dilution, filtration, purification, and the like.

All of the above compositions can be suitably used for forming the photosensitive layer in the manufacture of the photoelectric conversion element of the embodiment of the invention.

In the light absorbing agent composition and ammonium salt composition, a content b1 of the compound represented by $C(R^1)(R^2)(R^3)\text{-}N(R^{1a})_3X$ and a content b2 of the compound represented by $R^4\text{-}N(R^{2a})_3X$ each preferably satisfy a molar ratio defined by Formula (s1-1), more preferably a molar ratio defined by Formula (s1-2), even more preferably a molar ratio defined by Formula (s1-3), and particularly preferably a molar ratio defined by Formula (s1-4).

Formula (s1-1) $0.001 \leq b2/b1 \leq 9999$

Formula (s1-2) $4 \leq b2/b1 \leq 4999$

Formula (s1-3) $9 \leq b2/b1 \leq 1999$

Formula (s1-4) $9 \leq b2/b1 \leq 999$

Furthermore, in the formation of the photosensitive layer, a use amount b1 of the compound represented by $C(R^1)(R^2)(R^3)\text{-}N(R^{1a})_3X$, a use amount b2 of the compound represented by $R^4\text{-}N(R^{2a})_3X$, and a use amount c of the compound $MX_2$ are appropriately set within a range in which the perovskite compound can be formed. In the present invention, the contents b1, b2, and c preferably satisfy a molar ratio defined by Formula (s2-1), more preferably a molar ratio defined by Formula (s2-2).

Formula (s2-1) $0.8 \leq (b1 + b2)/c \leq 10$

Formula (s2-2) $0.9 \leq (b1 + b2)/c \leq 5$

In the formation of the photosensitive layer, a molar ratio, b1:b2:c, of the use amount b1 of the compound represented by $C(R^1)(R^2)(R^3)\text{-}N(R^{1a})_3X$, the use amount b2 of the compound represented by $R^4\text{-}N(R^{2a})_3X$, and the use amount c of the compound $MX_2$ can be set by appropriately combining each formula of Formulas (s1-1) to (s1-3), and each formula of Formulas (s2-1) and (s2-2).

Each concentration of solid contents of the light absorbing agent composition, the ammonium salt composition, and the metal salt composition is not particularly limited. For example, the concentration of solid contents of the light absorbing agent composition is preferably from 0.1% to 99% by mass, and more preferably from 1% to 70% by mass. The concentration of solid contents of the ammonium salt composition is preferably from 0.05% to 90% by mass, and more preferably from 0.1% to 55% by mass. The concentration of solid contents of the metal salt composition is preferably from 0.05% to 95% by mass, and more preferably from 0.1% to 80% by mass.

Examples of a method of providing the photosensitive layer 13 include a wet-type method and a dry-type method, and there is no particular limitation thereto. In the present invention, a wet-type method is preferable, and for example, a method in which the light absorbing agent composition or the ammonium salt composition are brought into contact with the surface of the layer forming the photosensitive layer 13 on the surface thereof (in the photoelectric conversion element 10, any layer of the porous layer 12, the blocking layer 14, the electron transport layer 15, or the hole transport layer 16) is preferable.

Specifically, as the method in which the light absorbing agent composition is brought into contact with the above surface, applying the light absorbing agent composition on the surface or immersing the composition is preferable. A contact temperature is preferably from 5°C to 100°C, and immersion time is preferably from 5 seconds to 24 hours and more preferably from 20 seconds to 1 hour. In a case of drying the light absorbing agent composition that is applied, with regard to the drying, drying with heat is preferable, and drying is performed by heating the applied light absorbing agent solution typically at 20°C to 300°C, and preferably at 50°C to 170°C.

In addition, the photosensitive layer can also be formed in conformity to a method of synthesizing the perovskite compound.

Preferable examples of a method of bringing the ammonium salt composition into contact with the surface include a method in which the ammonium salt composition and the metal salt composition are applied separately (including an immersion method) and dried if necessary. In this method, any composition may be applied to the surface first, but the metal salt composition is preferably applied to the surface first. Application conditions and drying conditions in this method are the same as those in the method of bringing the light absorbing agent composition into contact with the surface. In the method of bringing the ammonium salt composition into contact with the surface, instead of the application of the ammonium salt composition and the metal salt composition, the ammonium salt composition or the metal salt composition may be vapor-deposited.

Still another example of the method includes a dry-type method such as a vacuum deposition by using a mixture from which a solvent of the light absorbing agent composition is removed. For example, a method of simultaneously or sequentially vapor-depositing the compound AX and the compound $MX_2$ may be exemplified.

According to the methods and the like, the perovskite compound is formed on the surface of the porous layer 12, the blocking layer 14, the electron transport layer 15, or the hole transport layer 16 as the photosensitive layer.

The hole transport layer 3 or the electron transport layer 4 is preferably formed on the photosensitive layer 13 provided as described above.

The hole transport layer 3 can be formed through application and drying of a hole transporting material solution that contains a hole transporting material. In the hole transporting material solution, a concentration of the hole transporting material is preferably 0.1 to 1.0 M (mol/L) when considering that application properties are excellent, and in a case of providing the porous layer 12, the hole transporting material solution easily intrudes into pores of the porous layer 12.

The electron transport layer 4 can be formed through application and drying of an electron transporting material solution that contains an electron transporting material.

After the hole transport layer 3 or the electron transport layer 4 is formed, the second electrode 2 is formed, thereby manufacturing the photoelectric conversion element and the solar cell.

The film thicknesses of the respective layers can be adjusted by appropriately changing the concentrations of respective dispersion liquids or solutions and the number of times of application. For example, in a case where the photosensitive layers 13B and 13C having a large film thickness are provided, the light absorbing agent composition, or the ammonium salt composition or the metal salt composition may be applied plurality of times and dried.

The respective dispersion liquids and solutions described above may respectively contain additives such as a dispersion auxiliary agent and a surfactant as necessary.

Examples of the solvent or dispersion medium that is used in the method of manufacturing the photoelectric conversion element include a solvent described in JP2001-291534A, but the solvent or dispersion medium is not particularly limited thereto. In the present invention, an organic solvent is preferable, and an alcohol solvent, an amide solvent, a nitrile solvent, a hydrocarbon solvent, a lactone solvent, a halogen solvent, and a mixed solvent of two or more kinds thereof are preferable. As the mixed solvent, a mixed solvent of the alcohol solvent and a solvent selected from the amide solvent, the nitrile solvent, and the hydrocarbon solvent is preferable. Specifically, methanol, ethanol, isopropanol, γ-butyrolactone, chlorobenzene, acetonitrile, N,N'-dimethylformamide (DMF), dimethylacetamide, and a mixed solvent thereof are preferable.

A method of applying the solutions or dispersants which form the respective layers is not particularly limited, and it is possible to use a known application method such as spin coating, extrusion die coating, blade coating, bar coating, screen printing, stencil printing, roll coating, curtain coating, spray coating, dip coating, an inkjet printing method, and an immersion method. Among these, a spin coating method, a screen printing method, and the like are preferable.

The photoelectric conversion element of the embodiment of the invention may be subjected to an efficiency stabilizing treatment such as annealing, light soaking, and being left as is in an oxygen atmosphere as necessary.

The photoelectric conversion element prepared as described above can be used as a solar cell after connecting the external circuit 6 to the first electrode 1 and the second electrode 2.

Hereinafter, the present invention will be described in more detail based on examples, but the present invention is not limited thereto.

## EXAMPLES

The photoelectric conversion element 10A illustrated in Fig. 1 was manufactured in the following procedure. In a case where the film thickness of the photosensitive layer 13 is large, this case corresponds to the photoelectric conversion element 10B illustrated in Fig. 2.

[Manufacturing of photoelectric conversion element (sample No. 101)]

<Production of conductive support 11>

A fluorine-doped $SnO_2$ conductive film (transparent electrode 11b, film thickness: 300 nm) was formed on a

glass substrate (support 11a, thickness: 2.2 mm) to prepare the conductive support 11.

<Preparation of solution for blocking layer>

15% by mass isopropanol solution (manufactured by Sigma-Aldrich Co. LLC) of titanium diisopropoxide bis(acetylacetonate) was diluted with 1-butanol, thereby preparing 0.02 M solution for a blocking layer.

<Formation of blocking layer 14>

The blocking layer 14 (film thickness: 100 nm) formed from titanium oxide was formed on the $SnO_2$ conductive film of the conductive support 11 by using the prepared 0.02 M solution for the blocking layer at 450°C in accordance with a spray pyrolysis method.

<Preparation of titanium oxide paste>

Ethyl cellulose, lauric acid, and terpineol were added to an ethanol dispersion liquid of titanium oxide (anatase, average particle size: 20 nm), thereby preparing titanium oxide paste.

<Formation of porous layer 12>

The prepared titanium oxide paste was applied onto the blocking layer 14 with a screen printing method, and was baked. This titanium oxide paste was applied and baked twice, respectively. In regard to a baking temperature, first baking was performed at 130°C and second baking was performed at 500°C for 1 hour. A baked body of the titanium oxide, which was obtained, was immersed in 40 mM $TiCl_4$ aqueous solution, and was heated at 60°C for 1 hour, and heating was continuously performed at 500°C for 30 minutes, thereby forming the porous layer 12 (film thickness: 250 nm) formed from $TiO_2$.

<Formation of photosensitive layer 13A>

A methanol solution (27.86 mL) of 40% by mass of methyl amine, and an aqueous solution (30 mL) of 57% by mass of hydrogen iodide (hydriodic acid) were stirred in a flask at 0°C for 2 hours, and were concentrated, and therefore coarse $CH_3NH_3I$ was obtained. The coarse $CH_3NH_3I$ obtained was dissolved in ethanol and was recrystallized with diethylether. A precipitated crystal was filtered and collected and dried under reduced pressure at 60°C for 24 hours, and therefore purified $CH_3NH_3I$ (which is the compound represented by Formula (2b) and is the compound producing the organic cation (A2)) was obtained.

A 10% ethanol solution of the following compound b1 (100 g) and an aqueous solution of 57 mass% hydrogen iodide (38 g) were stirred in a flask at 0°C for 2 hours, and then the solvent was removed by concentration. The resultant product was further dried at 60°C for 24 hours under reduced pressure, and therefore the following compound c1 (which is the compound represented by Formula (1b) and is the compound producing the organic cation (A1)) was obtained.

Next, the purified $CH_3NH_3I$, the compound c1, and $PbI_2$ (halogenated metal) are mixed at a mixing molar ratio as follows by stirring in DMF at 60°C for 12 hours, and then filtered with a polytetrafluoroethylene (PTFE) syringe filter, and therefore a light absorbing agent solution A having a solid content of 40% by mass (ammonium salt composition containing the halogenated metal, moisture content: 0.1% by mass) was obtained.

$$\text{Purified } CH_3NH_3I/\text{compound c1} = 99$$

$$[\text{Purified } CH_3NH_3I + \text{compound c1}]/PbI_2 = 1.01$$

Purified $CH_3NH_3I$:compound c1:$PbI_2$ = 99:1:99.5

The prepared light absorbing agent solution A was applied (application temperature: 60°C) to the porous layer 12 by a spin coating method (2000 rpm, 60 seconds), and the applied light absorbing agent solution A was dried at 100°C for 90 minutes by a hot plate, and therefore a photosensitive layer 13A having the perovskite compound (having a film thickness of 300 nm (including a film thickness of 250 nm of the porous layer 12)) was formed.

In this manner, the first electrode 1 was produced.

<Preparation of hole transporting material solution>

Spiro-MeOTAD (180 mg) as the hole transporting material was dissolved in chlorobenzene (1 mL). An acetonitrile solution (37.5 μL) obtained by dissolving lithium-bis (trifluoromethanesulfonyl) imide (170 mg) in acetonitrile (1 mL) and t-butyl pyridine (TBP, 17.5 μL) were additionally mixed to the chlorobenzene solution, thereby preparing a hole transporting material solution.

<Formation of hole transport layer 3>

Next, the hole transporting material solution was applied on the photosensitive layer 13 of the first electrode 1 by a spin coating method, and the applied hole transporting material solution was dried, and therefore a hole transport layer 3 (having a film thickness of 0.1 μm) was formed.

<Production of second electrode 2>

Gold (film thickness of 0.1 μm) was deposited on the hole transport layer 3 by vapor deposition method, and therefore the second electrode 2 was produced.

In this manner, the photoelectric conversion element 10 of the sample No. 101 was manufactured.

Each film thickness was determined by observation with a scanning electron microscope (SEM).

[Manufacturing of photoelectric conversion elements (sample Nos. 102 to 146)]

Photoelectric conversion elements 10 of sample Nos. 102 to 146 were manufactured in the same manner as the manufacture of the photoelectric conversion element of the sample No. 101 except that a combination of the organic ammonium salt to be used and a mixing molar ratio were changed as shown in Table 1 in the manufacture of the photoelectric conversion element of the sample No. 101 described above.

b1
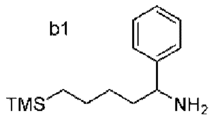
c1
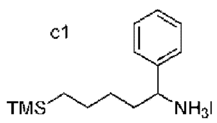
b12
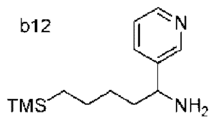
c12
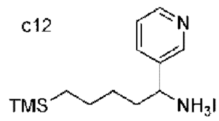

b2
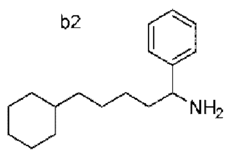
c2
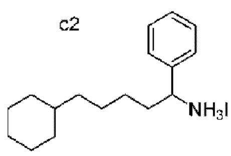
b13
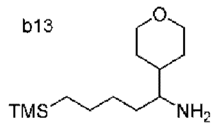
c13
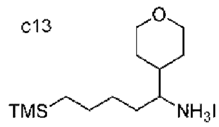

b6
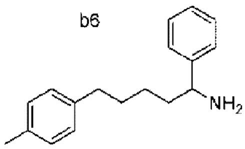
c6
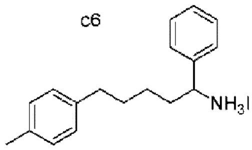
b14
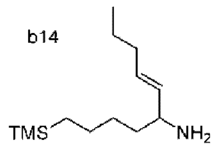
c14
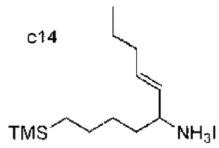

b7
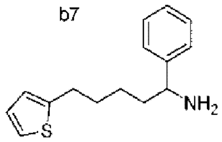
c7
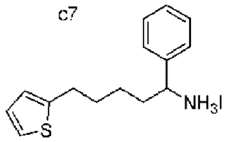
b15
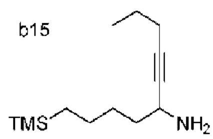
c15
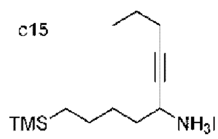

b8
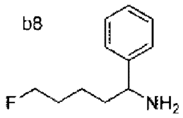
c8
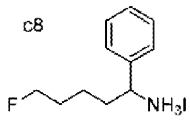
b16
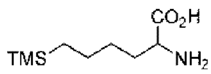
c16
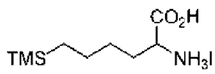

b9

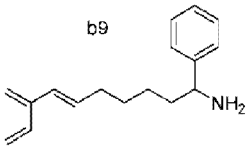

c9

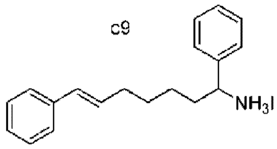

b18

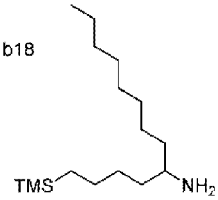

c18

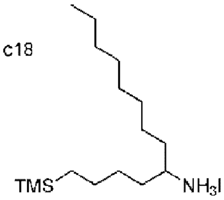

b10

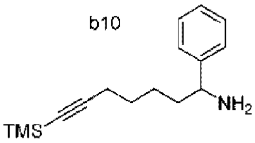

c10

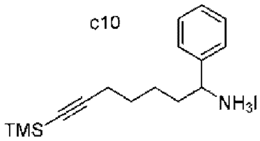

b19

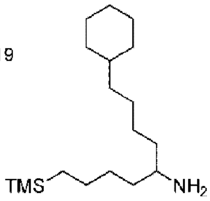

c19

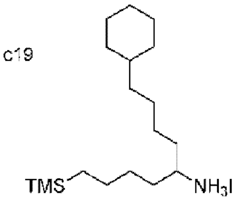

b11

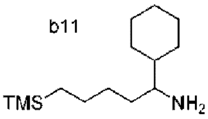

c11

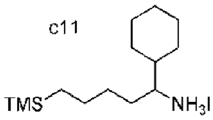

b20
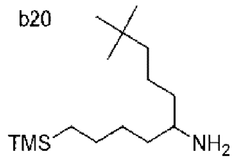

c20
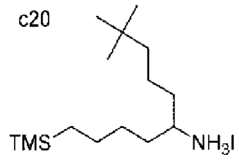

b32
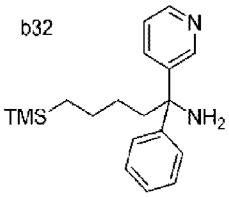

c32
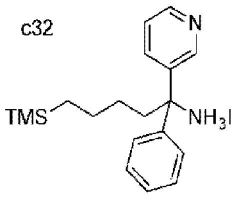

b33
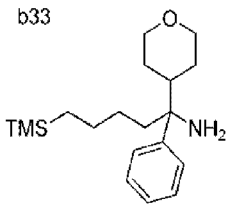

c33
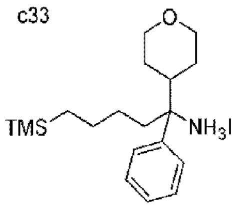

b34
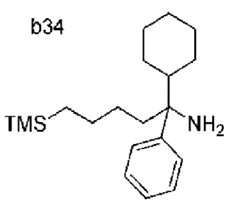

c34
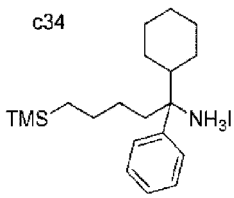

b35
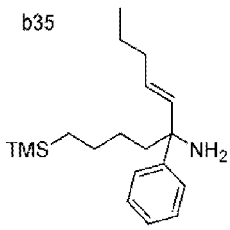

c35
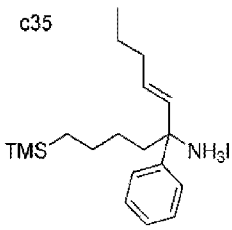

b36
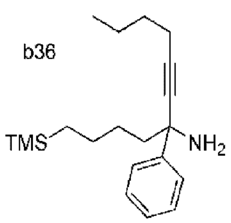

c36
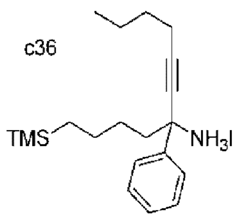

b37
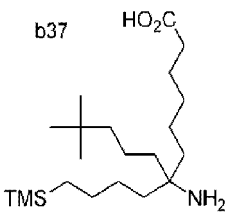

c37
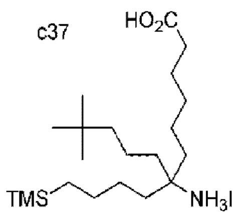

b38
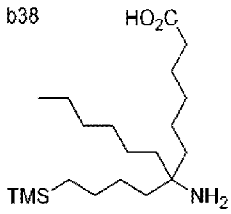

c38
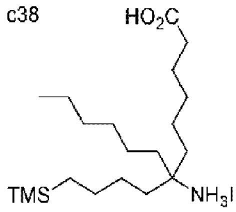

b39
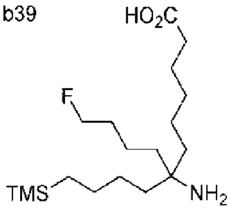

c39
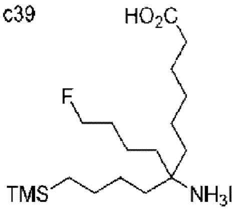

b40
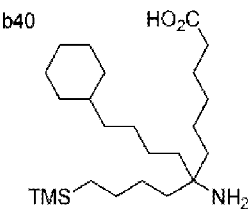

c40
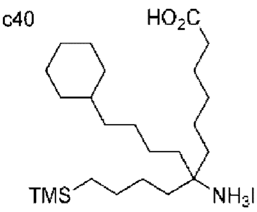

b41
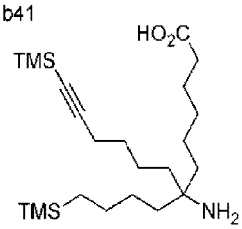

c41
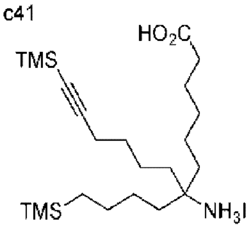

b42
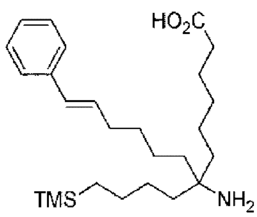

c42
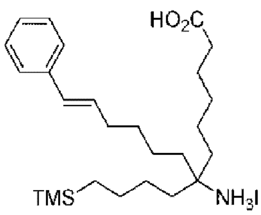

b44
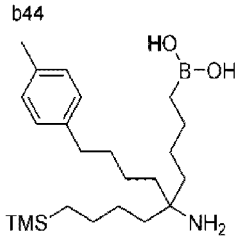

c44
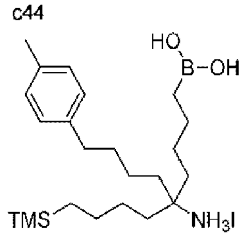

b45
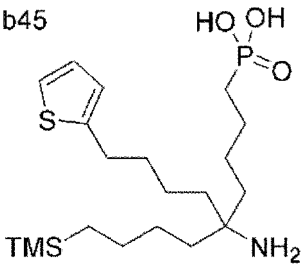

c45
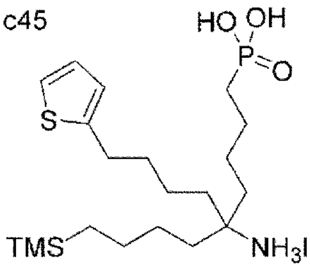

b47
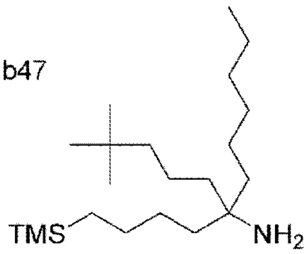

c47
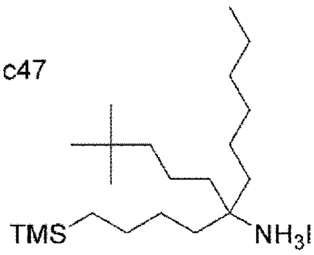

b48
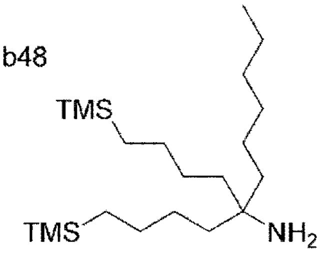

c48
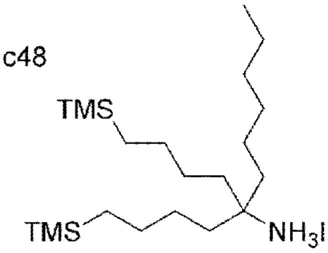

b49
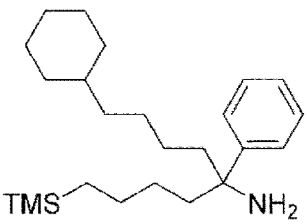

c49
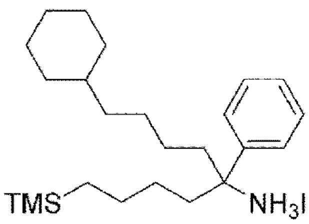

b50
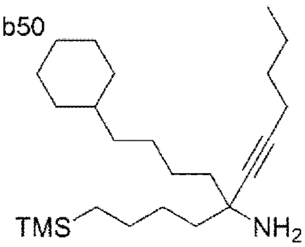

c50
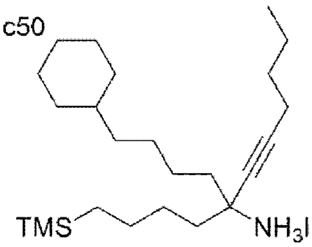

b51
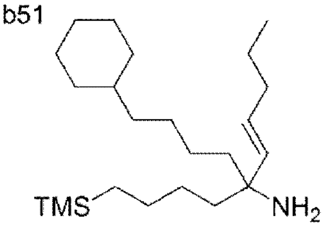

c51
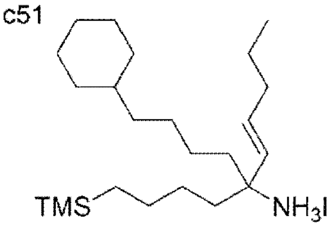

b52

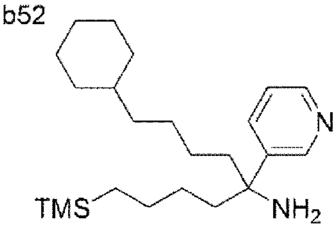

c52

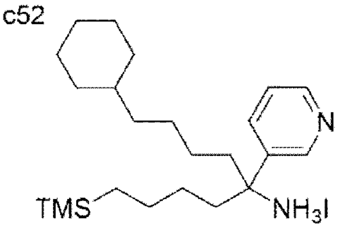

b53

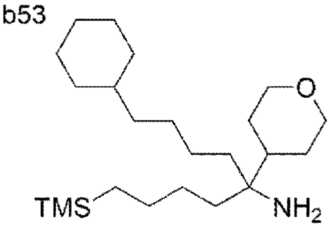

c53

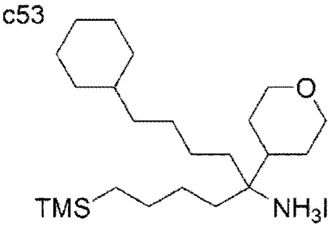

[Manufacturing of photoelectric conversion elements (sample Nos. c101 to c105)]

Photoelectric conversion elements 10 of the sample Nos. c101 to c105 were manufactured in the same manner as the manufacture of the photoelectric conversion element of the sample No. 101 except that the organic ammonium salt to be used and a use amount thereof were changed as shown in Table 1 in the manufacture of the photoelectric conversion element of the sample No. 101 described above.

In each of the photoelectric conversion elements of the embodiment of the invention manufactured as described above, the molar ratio ([A2]/[A1]) of the organic cation (A1) and the organic cation (A2) forming the perovskite-type crystal structure is practically the same value as a mixing molar ratio of the organic ammonium salt (compound of Formula (2b)/compound of Formula (1b)). In fact, in each of the photoelectric conversion elements of the sample Nos. 101 to 146, a molar ratio of the organic cation (A1) and the organic cation (A2) in the photosensitive layer was examined and found to be almost the same as the mixing molar ratio of the compound of Formula (1b) and the compound of Formula (2b). Measurement of the molar ratio was carried out using gas chromatography-mass spectrometry (GC-MS), X-ray diffraction (XRD), or liquid chromatography-mass spectrometry (LC-MS). In this case, if necessary, the sample was subjected to a base treatment, detected as an amine, or derivatized and detected.

[Evaluation of moisture resistance under high humidity]

Seven samples of each of the photoelectric conversion elements of each sample number were manufactured. A cell characteristic test was performed on the seven samples of the manufactured photoelectric conversion elements so as to obtain a photoelectric conversion efficiency. Measurement results were taken as an initial photoelectric conversion efficiency.

The cell characteristic test was performed by irradiating each photoelectric conversion element with pseudo-solar light of 1000 $W/m^2$ from a xenon lamp through an AM1.5 filter by using a solar simulator "WXS-85H" (manufactured by Wacom). The photoelectric conversion efficiency was obtained by measuring the current-voltage characteristics of each photoelectric conversion element irradiated with pseudo sunlight using a source meter "Keithley 2401" (manufactured by TEKTRONIX, INC.).

The Initial photoelectric conversion efficiency of each of the photoelectric conversion elements described above an efficiency with which the photoelectric conversion element or the solar cell functions sufficiently.

These samples were stored under conditions of 45°C and a humidity of 55% RH for 24 hours, and then the cell characteristic test was carried out again to obtain the photoelectric conversion efficiency. Measurement results were taken as a photoelectric conversion efficiency after a lapse of time. A reduction rate of the photoelectric conversion efficiency of each sample was calculated by the following formula.

Reduction rate = 1 - photoelectric conversion efficiency after a lapse of time/initial photoelectric conversion efficiency

For each of the photoelectric conversion elements of each sample number, an average of the reduction rates

of the seven samples was calculated. The calculated value was taken as the average reduction rate.

Next, in a case where the average reduction rate of the sample No. c101 is 1, a relative reduction rate of each example to the sample No. c101 (a value obtained by dividing the average reduction rate of each of the photoelectric conversion elements by the average reduction rate of the sample No. c101) was calculated, and evaluation was performed according to the following evaluation standard.

The evaluation of "E" or higher is practically required, and the evaluation of "D" or higher is preferable. The lower the relative reduction rate, the better the moisture resistance is.

-Evaluation standard of moisture resistance-

AA: The relative reduction rate is less than 0.65
A: The relative reduction rate is 0.65 or more and less than 0.70
B: The relative reduction rate is 0.70 or more and less than 0.75
C: The relative reduction rate is 0.75 or more and less than 0.80
D: The relative reduction rate is 0.80 or more and less than 0.85
E: The relative reduction rate is 0.85 or more and less than 0.90
F: The relative reduction rate is 0.90 or more and less than 0.95
G: The relative reduction rate is 0.95 or more

[Evaluation of stability of film form]

The stability of the film form was evaluated using the first electrode of the photoelectric conversion elements of each sample number (that is, in the manufacture of the photoelectric conversion elements of each sample number, the stability of the film form was evaluated using the sample of the aspect in which the hole transport layer and the second electrode are not formed).

Three samples of each of the first electrodes (25 mm square substrates) of the photoelectric conversion elements of each sample number were prepared and stored for 48 hours under the environment of a temperature of 25°C and a humidity of 50% RH. Thereafter, regarding the film form of the photosensitive layer of the first electrode, the surface shape and tint were respectively observed by SEM and visually, and the stability of the photosensitive layer was evaluated based on the following evaluation standard. The observation with a scanning electron microscope (SEM) was carried out at three points (n = 3) near the center of each sample at an acceleration voltage of 2 kV after each sample after the humidity test was coated with osmium (Os). The evaluation results of the three samples of the same composition were all the same. In the following evaluation A, the crystal structure of the surface of the photosensitive layer is not decomposed, and as going towards from the evaluation B to evaluation D, the crystal structure of the surface of the photosensitive layer is more decomposed.

-Evaluation standard for stability of film form-

The brownish-red mirror surface of the photosensitive layer is
A: Almost no change
B: White turbidity occurs and fine unevenness are formed on the film surface
C: Turning into gray, and unevenness is formed on the film surface
D: Turning into gray, and voids are formed in the film surface

[Table 1]

| Sample No. | Organic ammonium salt composition | | Compound of Formula (2b)/ Compound of Formula (1b)/] $PbI_2$ | Moisture resistance | Film form | |
|---|---|---|---|---|---|---|
| | Compound of Formula (1b) | Compound of Formula (2b) | | | | |
| 101 | c1 | $CH_3NH_3I$ | 99/1/99.5 | D | C | Present invention |
| 102 | c1 | $CH_3CH_2NH_3I$ | 99/1/99.5 | D | C | Present invention |

(continued)

| Sample No. | Organic ammonium salt composition | | Compound of Formula (2b)/ Compound of Formula (1b)/] $PbI_2$ | Moisture resistance | Film form | |
|---|---|---|---|---|---|---|
| | Compound of Formula (1b) | Compound of Formula (2b) | | | | |
| 103 | c1 | CH(=NH) $NH_3I$ | 99/1/99.5 | D | C | Present invention |
| 104 | c2 | $CH_3NH_3I$ | 99/1/99.5 | D | C | Present invention |
| 105 | c6 | $CH_3NH_3I$ | 99/1/99.5 | D | C | Present invention |
| 106 | c7 | $CH_3NH_3I$ | 99/1/99.5 | D | C | Present invention |
| 107 | c8 | $CH_3NH_3I$ | 99/1/99.5 | D | C | Present invention |
| 108 | c9 | $CH_3NH_3I$ | 99/1/99.5 | D | C | Present invention |
| 109 | c10 | $CH_3NH_3I$ | 99/1/99.5 | D | C | Present invention |
| 110 | c11 | $CH_3NH_3I$ | 99/1/99.5 | D | C | Present invention |
| 111 | c12 | $CH_3NH_3I$ | 99/1/99.5 | D | C | Present invention |
| 112 | c13 | $CH_3NH_3I$ | 99/1/99.5 | D | C | Present invention |
| 113 | c14 | $CH_3NH_3I$ | 99/1/99.5 | D | C | Present invention |
| 114 | c15 | $CH_3NH_3I$ | 99/1/99.5 | D | C | Present invention |
| 115 | c16 | $CH_3NH_3I$ | 99/1/99.5 | E | C | Present invention |
| 116 | c18 | $CH_3NH_3I$ | 99/1/99.5 | D | C | Present invention |
| 117 | c19 | $CH_3NH_3I$ | 99/1/99.5 | D | C | Present invention |
| 118 | c20 | $CH_3NH_3I$ | 99/1/99.5 | D | C | Present invention |
| 119 | c32 | $CH_3NH_3I$ | 99/1/99.5 | C | B | Present invention |
| 120 | c33 | $CH_3NH_3I$ | 99/1/99.5 | C | B | Present invention |
| 121 | c34 | $CH_3NH_3I$ | 99/1/99.5 | C | B | Present invention |
| 122 | c35 | $CH_3NH_3I$ | 99/1/99.5 | B | B | Present invention |
| 123 | c36 | $CH_3NH_3I$ | 99/1/99.5 | B | B | Present invention |

(continued)

| Sample No. | Organic ammonium salt composition | | Compound of Formula (2b)/ Compound of Formula (1b)/] $PbI_2$ | Moisture resistance | Film form | |
|---|---|---|---|---|---|---|
| | Compound of Formula (1b) | Compound of Formula (2b) | | | | |
| 124 | c47 | $CH_3NH_3I$ | 99/1/99.5 | B | B | Present invention |
| 125 | c48 | $CH_3NH_3I$ | 99/1/99.5 | B | B | Present invention |
| 126 | c49 | $CH_3NH_3I$ | 99/1/99.5 | B | B | Present invention |
| 127 | c50 | $CH_3NH_3I$ | 99/1/99.5 | B | B | Present invention |
| 128 | c51 | $CH_3NH_3I$ | 99/1/99.5 | B | B | Present invention |
| 129 | c52 | $CH_3NH_3I$ | 99/1/99.5 | B | B | Present invention |
| 130 | c53 | $CH_3NH_3I$ | 99/1/99.5 | B | B | Present invention |
| 131 | c37 | $CH_3NH_3I$ | 99/1/99.5 | AA | A | Present invention |
| 132 | c38 | $CH_3NH_3I$ | 99/1/99.5 | AA | A | Present invention |
| 133 | c39 | $CH_3NH_3I$ | 99/1/99.5 | AA | A | Present invention |
| 134 | c40 | $CH_3NH_3I$ | 999/1/999 | AA | A | Present invention |
| 135 | c40 | $CH_3NH_3I$ | 99/1/99.5 | AA | A | Present invention |
| 136 | c40 | $CH_3NH_3I$ | 95/5/97.5 | AA | A | Present invention |
| 137 | c40 | $CH_3NH_3I$ | 99/10/95 | AA | A | Present invention |
| 138 | c40 | $CH_3NH_3I$ | 99/1/104.5 | AA | A | Present invention |
| 139 | c40 | $CH_3CH_2NH_3I$ | 99/1/99.5 | AA | A | Present invention |
| 140 | c40 | $CH(=NH)NH_3I$ | 99/1/99.5 | AA | A | Present invention |
| 141 | c40 | $CH(=NH)NH_3I$ | 95/5/97.5 | AA | A | Present invention |
| 142 | c40 | $CH(=NH)NH_3I$ | 90/10/95 | AA | A | Present invention |
| 143 | c41 | $CH_3NH_3I$ | 99/1/99.5 | A | A | Present invention |
| 144 | c42 | $CH_3NH_3I$ | 99/1/99.5 | A | A | Present invention |

(continued)

| Sample No. | Organic ammonium salt composition | | Compound of Formula (2b)/ Compound of Formula (1b)/] $PbI_2$ | Moisture resistance | Film form | |
|---|---|---|---|---|---|---|
| | Compound of Formula (1b) | Compound of Formula (2b) | | | | |
| 145 | c44 | $CH_3NH_3I$ | 99/1/99.5 | AA | A | Present invention |
| 146 | c45 | $CH_3NH_3I$ | 99/1/99.5 | AA | A | Present invention |
| c101 | - | $CH_3NH_3I$ | 100/0/100 | G | D | Standard |
| c102 | - | $CH_3CH_2NH_3I$ | 100/0/100 | G | D | Comparative Example |
| c103 | - | $CH(=NH)NH_3I$ | 100/0/100 | G | D | Comparative Example |
| c104 | $CH_3(CH_2)_3NH_3I$ | $CH_3NH_3I$ | 99/1/99.5 | F | D | Comparative Example |
| c105 | $C_6H_5(CH_2)_2NH_3I$ | $CH_3NH_3I$ | 99/1/99.5 | F | D | Comparative Example |

As shown in Table 1, it was understood that, in the photoelectric conversion elements of the sample Nos. c101 to c 105 in which the crystal structure containing the organic cation of Formula (1) was not formed in the photosensitive layer, the crystal structure constituting the photosensitive layer was likely to be damaged in a high-humidity environment, and the obtained photoelectric conversion elements had inferior moisture resistance.

On the other hand, in the photoelectric conversion elements of the sample Nos. 101 to 146 in which the crystal structure containing the organic cation of Formula (1) was formed in the photosensitive layer, the damage on the crystal structure constituting the photosensitive layer was efficiently prevented even under a high-humidity environment, and the obtained photoelectric conversion elements had excellent moisture resistance.

While the present invention has been described together with the embodiments thereof, any detail in the descriptions is not intended to limit the invention unless otherwise specified and is perceived to be interpreted widely without infringing the spirit and scope of the invention as set forth in the appended claims.

Priority is claimed on JP2016-168769 filed on August 31, 2016 in Japan, the content of which is incorporated in the present specification by reference as a part.

EXPLANATION OF REFERENCES

1A to 1F: first electrode
11: conductive support
11a: support
11b: transparent electrode
12: porous layer
13A to 13C: photosensitive layer
14: blocking layer
2: second electrode
3A, 3B, 16: hole transport layer
4, 15: electron transport layer
6: external circuit (lead)
10A to 10F: photoelectric conversion element
100A to 100F: system using solar cell
M: electric motor

## Claims

1. A photoelectric conversion element, comprising:

a first electrode that includes a photosensitive layer containing a light absorbing agent on a conductive support; and

a second electrode that is opposite to the first electrode,

wherein the light absorbing agent contains a compound having a perovskite-type crystal structure that has an organic cation, a cation of a metal atom, and an anion, and

the organic cation includes an organic cation represented by Formula (1) and an organic cation represented by Formula (2):

Formula (1) 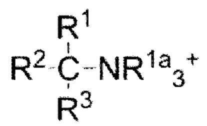

in Formula (1), $R^1$ and $R^2$ represent a group selected from a substituent group Z below, $R^3$ represents a hydrogen atom or a group selected from the substituent group Z below, and $R^{1a}$ represents a hydrogen atom or a substituent, provided that not all of $R^1$, $R^2$ and $R^3$ are acidic groups:

[substituent group Z]: a group represented by Formula (1a), an aryl group, a cycloalkyl group, a heteroaryl group, an aliphatic heterocyclic group, an alkenyl group, an alkynyl group, and an acidic group

Formula (1a) $R^{1b}—(CR^{1c}_2)_n—*$

in Formula (1a), $R^{1b}$ represents a methyl group, a silyl group, a cycloalkyl group, an aryl group, a heteroaryl group, an aliphatic heterocyclic group, a halogen atom, or an acidic group, $R^{1c}$ represents a hydrogen atom or represents a substituent other than an alkenyl group and other than an alkynyl group, n represents integer of 3 to 15, and * represents a binding site with a carbon atom represented in Formula (1),

Formula (2) $R^4\text{-}NR^{2a}_3{}^+$

in Formula (2), $R^4$ represents a methyl group, an ethyl group, or a group represented by Formula (2a), and $R^{2a}$ represents a hydrogen atom, an alkyl group, a cycloalkyl group, an alkenyl group, an alkynyl group, an aryl group, a heteroaryl group, or an aliphatic heterocyclic group,

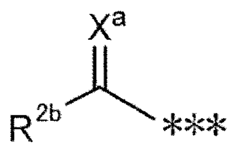

Formula (2a)

in Formula (2a), $X^a$ represents $NR^{2c}$, an oxygen atom, or a sulfur atom, $R^{2b}$ and $R^{2c}$ represent a hydrogen atom or a substituent, and *** represents a binding site with a nitrogen atom represented in Formula (2).

2. The photoelectric conversion element according to claim 1,

wherein $R^1$ and $R^2$ represent a group selected from a group represented by Formula (1a), an aryl group, a cycloalkyl group, a heteroaryl group, an aliphatic heterocyclic group, an alkenyl group, and an alkynyl group, and $R^3$ represents a hydrogen atom or a group selected from the substituent group Z.

3. The photoelectric conversion element according to claim 1 or 2, wherein all of $R^1$, $R^2$, and $R^3$ represent a group selected from a group represented by Formula (1a), an aryl group,

a cycloalkyl group, a heteroaryl group, an aliphatic heterocyclic group, an alkenyl group, and an alkynyl group.

**4.** The photoelectric conversion element according to claim 3, wherein $R^1$ and $R^2$ represent a group selected from a group represented by Formula (1a), an alkenyl group, and an alkynyl group.

**5.** The photoelectric conversion element according to claim 4,

wherein $R^1$ and $R^2$ represent a group represented by Formula (1a), and
$R^{1b}$ in $R^1$ represents a methyl group, a silyl group, a cycloalkyl group, an aryl group, or a halogen atom, and
$R^{1b}$ in $R^2$ represents an acidic group.

**6.** The photoelectric conversion element according to claim 5,

wherein $R^{1b}$ in $R^1$ represents a methyl group, a silyl group, or a cycloalkyl group, and
$R^{1b}$ in $R^2$ represents an acidic group.

**7.** The photoelectric conversion element according to claim 5 or 6, wherein all of $R^1$, $R^2$, and $R^3$ represent a group represented by Formula (1a).

**8.** A solar cell which is formed of the photoelectric conversion element according to any one of claims 1 to 7.

FIG. 1

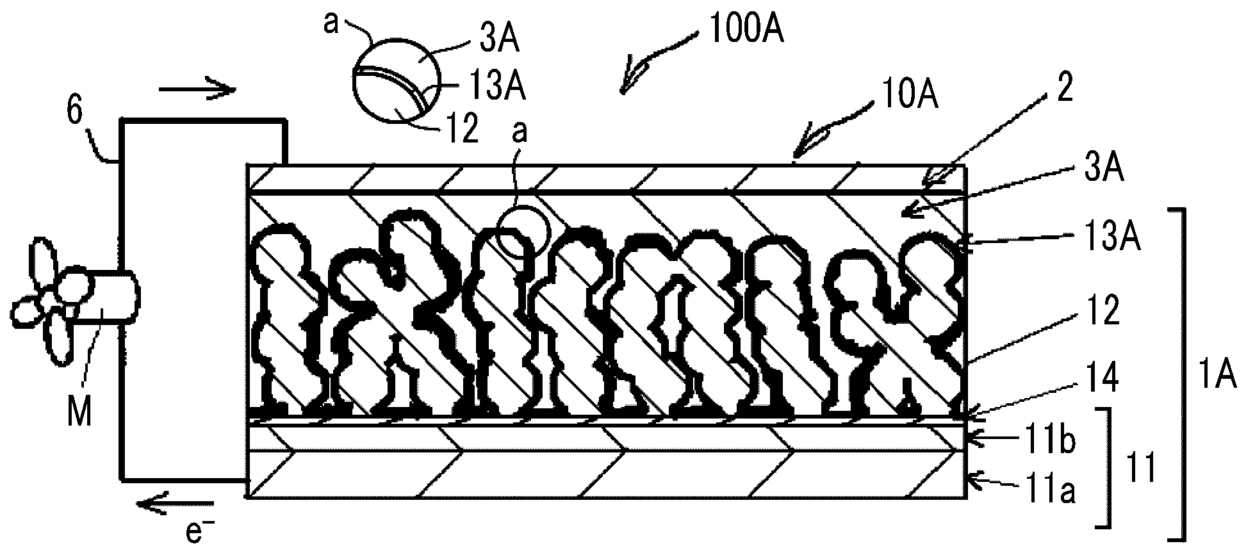
a
3A
13A
12
100A
6
10A
2
a
3A
13A
12
14
1A
11b
11
11a
M
e⁻

FIG. 2

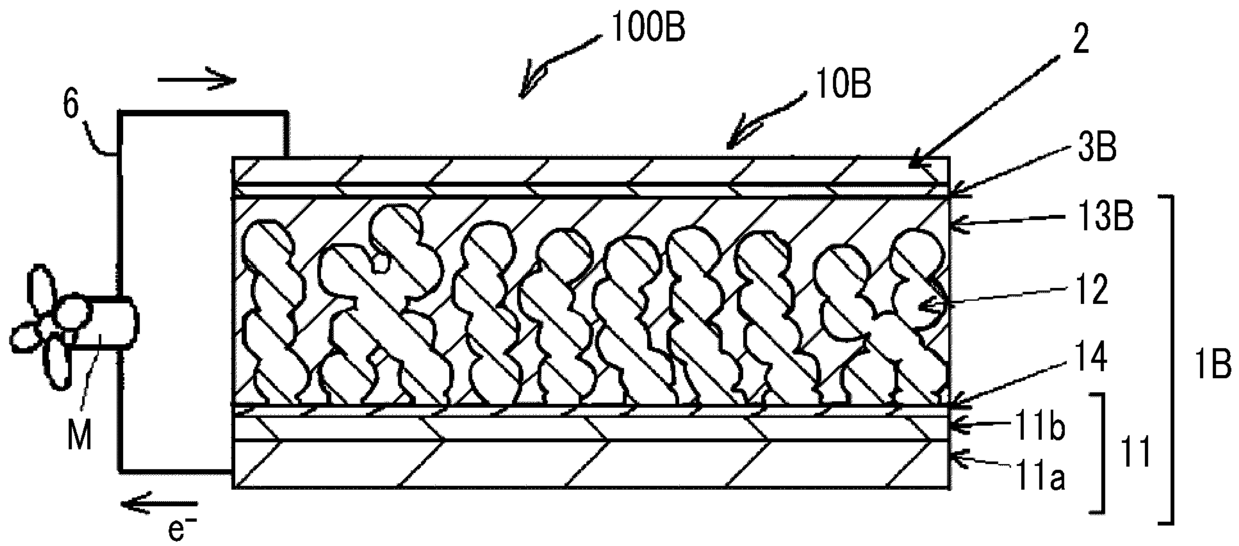
100B
2
6
10B
3B
13B
12
14
1B
11b
11
11a
M
e⁻

FIG. 3

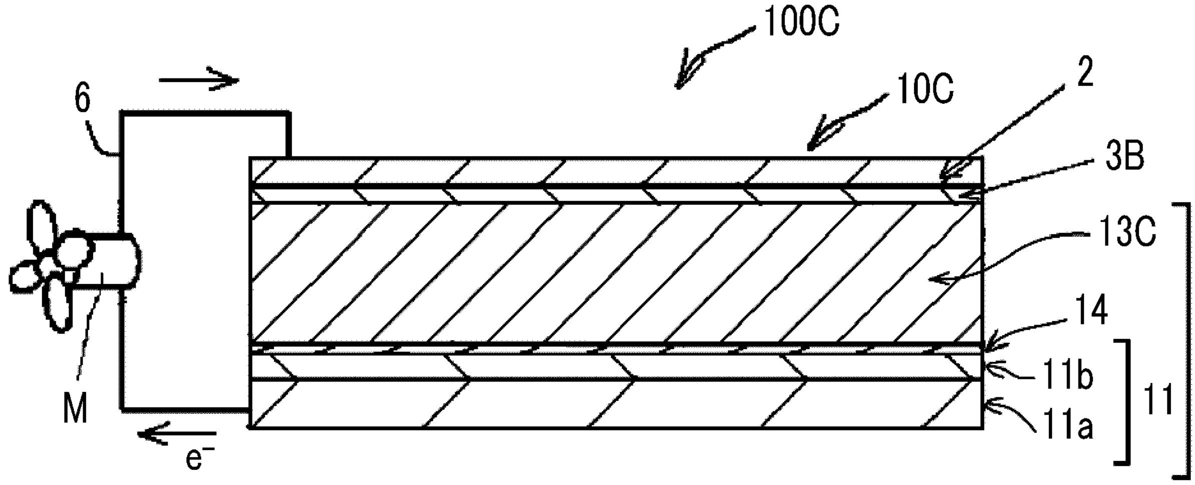
100C
10C
2
3B
6
13C
14
1C
11b
11
11a
M
e⁻

FIG. 4

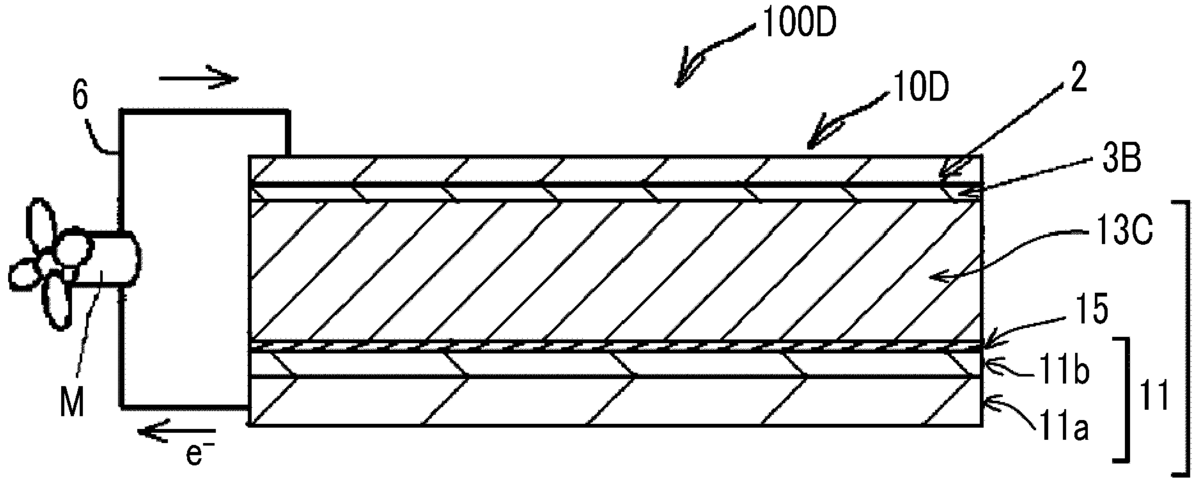
100D
10D
2
3B
6
13C
15
1D
11b
11
11a
M
e⁻

FIG. 5

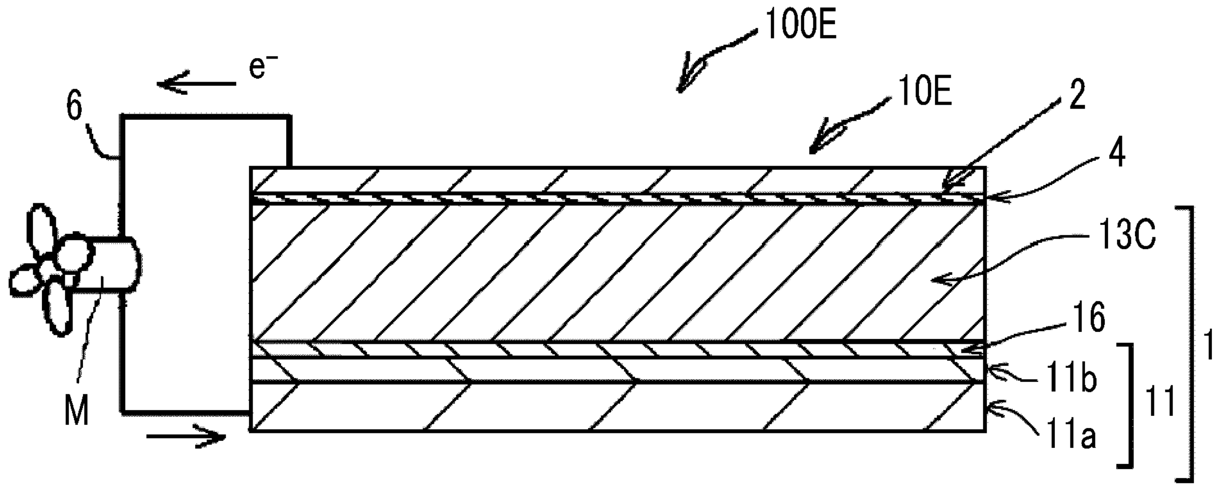
100E
e⁻
6
10E
2
4
13C
16
1E
11b
11
M
11a

FIG. 6

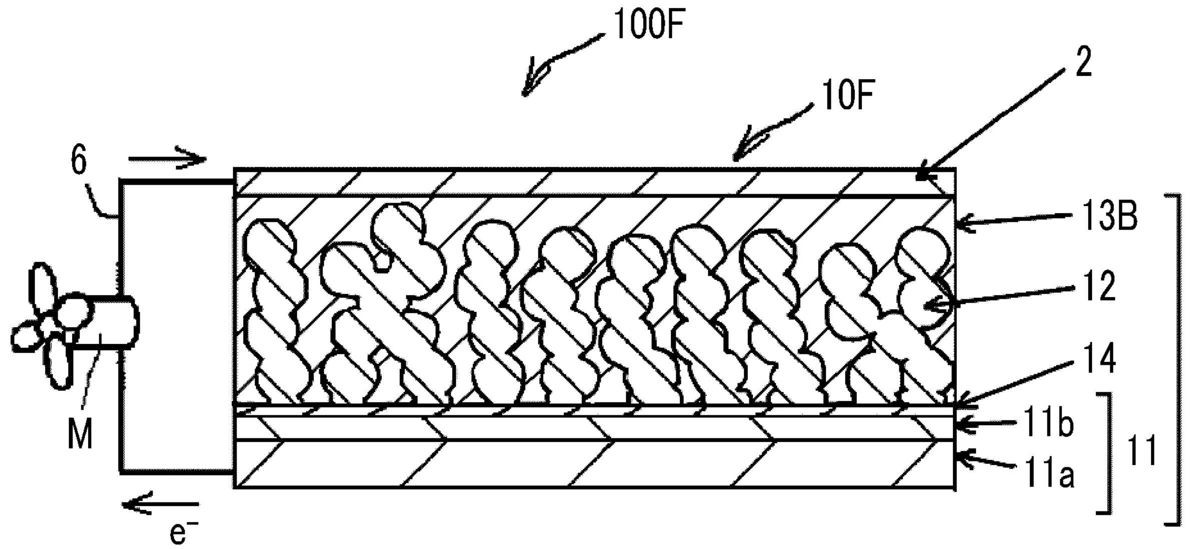
100F
2
10F
6
13B
12
14
1F
11b
M
11
11a
e⁻

## INTERNATIONAL SEARCH REPORT

International application No.
PCT/JP2017/030699

A. CLASSIFICATION OF SUBJECT MATTER
*H01L51/44*(2006.01)i, *H01G9/20*(2006.01)i

According to International Patent Classification (IPC) or to both national classification and IPC

B. FIELDS SEARCHED

Minimum documentation searched (classification system followed by classification symbols)
H01L51/42-51/48

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2017 |
| Kokai Jitsuyo Shinan Koho | 1971-2017 | Toroku Jitsuyo Shinan Koho | 1994-2017 |

Electronic data base consulted during the international search (name of data base and, where practicable, search terms used)
CAplus/REGISTRY(STN), Wiley Online Library

C. DOCUMENTS CONSIDERED TO BE RELEVANT

| Category* | Citation of document, with indication, where appropriate, of the relevant passages | Relevant to claim No. |
|---|---|---|
| A | WO 2016/005868 A1 (ECOLE POLYTECHNIQUE FEDERALE DE LAUSANNE (EPFL)), 14 January 2016 (14.01.2016), & JP 2017-520931 A & US 2017/0162811 A1 & EP 2966703 A1 & KR 10-2017-0030625 A & CN 106716664 A | 1-8 |
| A | WO 2015/159952 A1 (Fujifilm Corp.), 22 October 2015 (22.10.2015), & US 2017/0033299 A1 & EP 3133658 A1 & CN 106233484 A | 1-8 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 08 November 2017 (08.11.17) | 21 November 2017 (21.11.17) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

**INTERNATIONAL SEARCH REPORT**

International application No.
PCT/JP2017/030699

C (Continuation). DOCUMENTS CONSIDERED TO BE RELEVANT

| Category* | Citation of document, with indication, where appropriate, of the relevant passages | Relevant to claim No. |
|---|---|---|
| A | Ian C. Smith et al., "A Layered Hybrid Perovskite Solar-Cell Absorber with Enhanced Moisture Stability", Angewandte Chemie, 2014.09.01, Vol.126, p.11414-11417 [online], [retrieved on 2017.11.08], Retrieved from the Internet: <URL: http://onlinelibrary.wiley.com/doi/10.1002/ange.201406466/full><DOI:10.1002/ange.201406466> | 1-8 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2001291534 A
- US 4927721 A
- US 4684537 A
- US 5084365 A
- US 5350644 A
- US 5463057 A
- US 5525440 A
- JP 7249790 A
- JP H7249790 A
- JP 2004220974 A
- JP 2008135197 A
- JP 2005135902 A
- JP 2001160425 A
- JP 2003123859 A
- JP 2002260746 A
- JP 2016168769 A

### Non-patent literature cited in the description

- *Science,* 2012, vol. 338, 643-647
- *Angew. Chem. Int. Ed.,* 2014, vol. 53, 11232-11235
- *Science,* 2014, vol. 345, 295
- *J. Am. Chem. Soc.,* 2009, vol. 131 (17), 6050-6051
- *Science,* 2012, vol. 338, 643
- **AKIHIRO KOJIMA ; KENJIRO TESHIMA ; YASUO SHIRAI ; TSUTOMU MIYASAKA.** Organometal Halide Perovskites as Visible-Light Sensitizers for Photovoltaic Cells. *J. Am. Chem. Soc.,* 2009, vol. 131 (17), 6050-6051
- *Chemical Review,* 2010, vol. 110, 6595